# EUROPEAN PATENT APPLICATION

(11) **EP 4 454 666 A1**
(43) Date of publication of application: **30.10.2024**
(21) Application number: 23169614.7
(22) Date of filing: 24.04.2023
(51) Int. Cl.: A61K 51/10, A61K 103/00

(54) **IN VIVO IMAGING OF AN ALS BIOMARKER AND MEANS THEREFOR**

(71) Applicant: Société de Commercialisation des Produits de la Recherche Appliquée - SOCPRA Sciences Santé et Humaines S.E.C., Sherbrooke, QC J1L 1E2 (CA)
(72) Inventor: GUÉRIN, Brigitte, J0B 1R0 Westbury, Québec (CA); TREMBLAY, Sébastien, J1J 1K8 Sherbrooke, Québec (CA); AIT-MOHAND, Samia, J1N 2Z7 Sherbrooke, Québec (CA); SALZMANN, Michael, 8032 Zürich (CH); MAIER, Marcel, 8049 Zürich (CH)
(74) Representative: Witthoff Jaekel Steinecke Patentanwälte PartG mbB

(57) **Abstract**

Provided are labeled antibody conjugates for use in a method of diagnosing ALS or monitoring drug therapy and/or progression of ALS by *in vivo* detecting and tracing misfolded SOD1 by immunoimaging in a subject, compositions and kits comprising said conjugates and methods of *in vivo* immunoimaging of superoxide dismutase (SOD1) useful as a marker of amyotrophic lateral sclerosis (ALS) or efficacy of drug therapy of ALS in subject using these conjugates. Furthermore, SOD1-antibodies specific for misfolded and/or aggregated SOD1 are provided for use in treatment of bone and/or joint related diseases.

## Description

### FIELD OF THE INVENTION

The invention relates to means for *in vivo* immunoimaging of misfolded SOD1 (mSOD1). More specifically, the present invention relates to antibodies and antibody conjugates, respectively, which are useful in the diagnosis and in monitoring treatment/progression of diseases due to or associated with misfolded and aggregated SOD1, in particular neurogenerative diseases such as amyotrophic lateral sclerosis (ALS). Furthermore, the invention relates to anti-SOD1 antibodies for use in targeting SOD1 in the cartilage, for example at bones and joints in subjects.

### BACKGROUND OF THE INVENTION

ALS is a rare neurodegenerative disease resulting from the loss of motor neurons in the motor cortex, brainstem, and spinal cord. It currently affects three of every 100,000 people in the US and approximately 5,000 people are diagnosed with ALS each year. The majority of patients are between 40 and 70 years of age (ALSA 2019). The typically rapid decline in neurologic function results from the death of motor neurons that enable movement, speech, swallowing and breathing. Effective denervation of these muscles results in wasting and atrophy with patients eventually requiring assisted ventilation before succumbing to respiratory failure, which is the most common cause of death in ALS (Tateno *et al.* 2014).

Of all ALS cases ~10% are inherited (FALS) and the rest arise sporadically (SALS). Mutations in the SOD1 gene, leading to the misfolding of its protein product, are the second most common genetic cause of ALS (Vu and Browser 2017). While the exact mechanism of mutant-SOD1 toxicity is still unknown, most evidence points to a pathological gain of function associated with the propensity of this protein to misfold and aggregate (Li and Cashman 2014, Bosco *et al.* 2010, Forsberg *et al.* 2011, Forsberg *et al.* 2010, Graffmo *et al.* 2013).

Preclinical disease modeling utilizing transgenic mice (Tg) has generated data supporting the hypothesis that misfolding of wild-type non mutated SOD1 protein occurs and can also generate a "toxic conformation" that results in an ALS like pathology (Ayers *et al.* 2016, Bidhendi *et al.* 2016). Detection of misfolded wild-type SOD1 within human post-mortem sporadic ALS samples has been used to support the hypothesis that misfolded forms of wild-type SOD1 can contribute to sporadic ALS pathogenesis (Bosco *et al.* 2010, Forsberg *et al.* 2011, Forsberg *et al.* 2010, Tokuda *et al.* 2019, Pare *et al.* 2018, Maier *et al.* 2018).

Preliminary data was generated using the CSF from a cohort of SALS patients, where results suggested that 60-70% of SALS patients were also positive for misfolded SOD 1. Overall, these results along with IHC (immunohistochemistry) data suggest that the full penetrance of expression of misfolded SOD1 in these patients is significantly higher than detection from CSF would predict. Implications for this include the assumption that while the CSF assay under development is potentially a useful tool to support diagnosis of ALS, the assay does not yet provide a suitable prospective patient selection or diagnostic tool for the identification of pre-symptomatic ALS. Furthermore, an assay that relies on the detection of misfolded SOD1 in the CSF may not be suitable for all patients, as there may be factors that influence the appearance of misfolded SOD1 in the CSF vs the spinal tissue of patients.

Currently, there is no approved diagnostic tool for ALS, resulting in misdiagnosis and significant disease progression before formal diagnosis. The diagnosis of ALS is based thus on a symptom analysis including full medical history of a patient and requires time, because usually a neurologic examination is conducted at regular intervals to assess whether symptoms such as muscle weakness, atrophy of muscles, hyperreflexia, and spasticity are worsening. A non-invasive imaging test for early detection of ALS and for monitoring disease progression would have significant diagnostic and prognostic value thus. To date, there is also no cure for ALS. Only two symptomatic treatments have been approved by the FDA with minor effects on disease progression; and successful attenuation of disease progression requires early and accurate diagnosis to enable patient access to these standard of care therapies.

Therefore, there is an urgent need for diagnostic tools, in particular for tools usable *in vivo,* which could be used for early detection of misfolded SOD1 as indicator of ALS or for monitoring ALS progression or its treatment.

### SUMMARY OF THE INVENTION

The present invention generally relates to an antibody and equivalent binding molecules, which are conjugated to a detectable label and allow *in vivo* immunoimaging of misfolded and/or aggregated SOD1 in a subject. The labeled antibody conjugate is particularly useful in a method of diagnosing ALS and/or monitoring the therapy and/or progression of ALS in a subject. The method preferably comprises the detection of misfolded SOD1 by *in vivo* immunoimaging in the subject. The antibody and equivalent binding molecule specifically bind to misfolded and/or aggregated SOD1 and is conjugated to a detection label. Preferably, the subject is human.

The present invention is based on a recombinant human monoclonal antibody (AP-101 described Maier et al., Sci Transl. Med. (2018):10 (470)) that selectively targets mSOD1, which is coupled to a PET imaging agent, featuring⁸⁹Zr-DFO-AP-101 radiotracer for PET imaging of mSOD1.

As illustrated in Example 1 and in the preceding Materials and Methods section, DFO-AP-101 was prepared by conjugating AP-101 antibody with *p*-SCN-Bn desferoxamine *p*-SCN-Bn desferoxamine (SCN-Bn-DFO) and labelled with ⁸⁹Zr (t½=78.41h). A longitudinal imaging study was performed to identify the optimal mice age and time post administration of ⁸⁹Zr-DFO-AP-101 for the detection mSOD1 aggregation in transgenic (Tg, [B6.Cg-Tg(SOD1^{∗}G93A)1Gur/J)]) mice expressing mSOD1 and in wild type (Wt) mice. As control, a subset of mice were co-injected with AP-101 (100 mg/kg) to assess target specificity. PET/CT data were expressed as percent injected dose per gram of tissue (%ID/g).

As demonstrated in Example 4, ⁸⁹Zr-DFO-AP-101 was able to engage mSOD1 aggregates in the spinal cord of Tg mice and imaging was optimal in 126-day-old Tg mice and at day 10 post administration. The number of detected aggregates was more important at day 10 (18) as compared to day-7 (6). All the spots found between the T10 and L2 vertebra. The concentration of ⁸⁹Zr-DFO-AP-101 in the spinal cord and vertebra of the Tg mice significantly exceeded that of the Wt mice (p = 0.01). Co-injection with AP-101 considerably reduced the amount of mSOD1 aggregates detected from 67% (6/9) to 15% (5/13). The intensity of the aggregates was also decreased from 8.05 ± 1.30 %ID/g to 5.73 ± 0.28 with ⁸⁹Zr-DFO-AP-101 alone or in presence of AP-101 in excess. Blocking with AP-101, significantly reduced the spinal cord (p = 0.0002) and vertebra (p < 0.0001) uptake of ⁸⁹Zr-DFO-AP-101.

In addition, as shown in Example 7, in context with those experiments it was discovered that misSOD1 can also be found in the bone/joints of the SOD1 animals (PET signal, confirmed by IHC with other misSOD1 antibodies).

In summary, labeled anti-SOD1 antibody conjugates exemplified by the lead ⁸⁹Zr-DFO-AP-101 exhibit unprecedented detection of mSOD1 aggregates in transgenic mice. Accordingly, such labelled antibody conjugates find application in the early diagnostic of ALS and in the monitoring of therapeutic interventions. In addition, such construct but also mSOD1-binding molecules such as anti-SOD1 antibodies in general can be used for targeting SOD1, *i.e.,* misfolded and aggregated SOD1, respectively, at bones and/or joints and may open up a path of potential treatment/improvement of "bone problems" of ALS patients in the future

Accordingly, the present invention generally relates to antibodies and equivalent SOD1 binding molecules for use in the detection of misfolded and aggregated SOD1 *in vivo,* preferably immunoimaging of misfolded and/or aggregated SOD1 in a subject. The imaging method for detecting the antibody-label conjugate in the subject is preferably selected from the group comprising or consisting of PET, SPECT, MRI, MPI and optical imaging including fluorescence imaging, or combinations thereof or combinations with CT, such as PET/CT, PET/MRI, PET/fluorescence, SPECT/CT imaging.

The label for the antibody conjugate or equivalent binding molecule is selected depending on the imaging method intended to be used for diagnosing ALS or monitoring drug therapy and/or progression of ALS in a subject. If a use in PET and/or SPECT is intended, preferably the label is a radionuclide or a chemical compound (chelator or prosthetic group) in which one or more atoms have been replaced by a nuclide. If a use only in PET is intended, preferably a nuclide is selected, which emits positrons. If a use only in SPECT is intended, preferably a nuclide is selected which emits gamma rays. If a use in either PET or alternatively in SPECT is intended, preferably the antibody or equivalent binding molecule is conjugated at the same time to a prosthetic group and to a chelator. By this, a PET label, *e.g.,* a nuclide emitting positrons (*e*.*g*., F-18) can be conjugated to the prosthetic group and a SPECT label, *e.g.,* a nuclide emitting gamma rays (*e.g.,* Ga-67) can be complexed via the chelator. In one embodiment the antibody or equivalent binding molecule can be conjugated at the same time to a prosthetic group, for labelling with a PET label as exemplarily listed hereinbelow, and to a chelator to bind a SPECT label as exemplarily listed hereinbelow.

Preferably, the label to be included in the labeled antibody conjugate or labeled equivalent binding molecule is selected from: Fluorine-18 (F-18), Titanium-45 (Ti-45), Manganese-52 (Mn-52), Iron-52 (Fe-52), Kalium-43 (K-43), Scandium-43 (Sc-43), Scandium-44 (Sc-44), Cobalt-57 (Co-57), Copper-60 (Cu-60), Copper-61 (Cu-61), Copper-62 (Cu-62), Copper-64 (Cu-64), Copper-67 (Cu-67), Gallium-67 (Ga-67), Gallium-68 (Ga-68), Bromine-76 (Br-76), Bromine-77 (Br-77), Krypton-81m (Kr-81m), Rubidium-81 (Rb-81), Yttrium-86 (Y-86), Strontium-87m (Sr-87m), Zirconium-89 (Zr-89 or 89Zr), Technetium-99m (Tc-99m), Indium-111 (In-111), Indium-113m (In-113m), Antimony-117 (Sb-117), Iodine-123 (I-123), Iodine-125 (I-125), Caesium-127 (Cs-127), Caesium-129 (Cs-129), Iodine-131 (1-131), Iodine-132 (I-132), Lutetium-177 (Lu-177), Rhenium-186 (Re-186), Quicksilver-197 (Hg-197), Rhenium-188 (Re-188), Lead-203 (Pg-203), Bismuth-206 (Bi-206), Actinium-225 (Ac-225), Radium-225 (Ra-225) and a combination of any two or more thereof.

More preferred, the label is selected from the group comprising or consisting of:
(a) for use in PET from: Fluorine-18 (F-18), Manganese-52 (Mn-52), Copper-64 (Cu-64), Gallium-67 (Ga-67), Gallium-68 (Ga-68), Antimony-117 (Sb-117), Scandium-43 (Sc-43), Scandium-44 (Sc-44), Titanium-45 (Ti-45), Bromium-76 (Br-76), Yttrium-86 (Y-86), Iodine-124 (I-124) and Zirconium-89 (Zr-89 or ⁸⁹Zr);
(b) for use in SPECT from: Indium-111 (In-111), Technetium-99m (Tc-99m), Iodine-123 (I-123), Iodine-131 (1-131), Lutetium-177 and Rhenium-186 (Re-186);
(c) for use in MRI and MPI: magnetic, paramagnetic or superparamagnetic ion complexes or particles, preferably containing Gaddolinum³⁺ (Gd³⁺), Manganese²⁺ (Mn²⁺); Copper ²⁺ (Cu²⁺); iron oxide (Fe₃O₄) contrast agents; and
(d) for use in optical imaging including fluorescent imaging from: fluorescein, Cy2, Cy3, Cy3B, Cy3.5, Cy5, Cy5.5, Cy7, Texas Red^{®} (Molecular Probes, Inc., Eugene, OR), AlexaFluor^{®} (Molecular Probes, Inc., Eugene), AlexaFluor 350, AlexaFluor 405, AlexaFluor 430, AlexaFluor 488, AlexaFluor 500, AlexaFluor 532, AlexaFluor 546, AlexaFluor 568, AlexaFluor 594, AlexaFluor 610, AlexaFluor 633, AlexaFluor 647, AlexaFluor 660, AlexaFluor 680, AlexaFluor 700, AlexaFluor 750, BODIPY FL, BODIPY R6G, BODIPY TMR, BOPDIPY 530/550, BODIPY 558/568, BODIPY 564/570, BODIPY 576/589, BODIPY 581/591, BODIPY 630/650, BODIPY 650/665, Rhodamine Green^{™}-X (Molecular Probes, Inc.), Rhodamine Red^{™}-X (Molecular Probes, Inc.), Rhodamine 6G, TMR, TAMRA^{™} (Applied Biosystems).

More preferred, the detection label is a radionuclide. All radionuclides that may be detected in positron emission tomography (PET) and/or in single photon emission computed tomography (SPECT) can be used according to the present invention and thereby be part of the detection label, however, preferably the physical half-life of a radionuclide should match its intended application, or more specifically, the biologic half-life of the vector, *i*.*e*., antibody it is conjugated to.

Radionuclide tracers have an energy of from about 20 to about 4,000 kiloelectronvolts (*i.e.,* keV). For SPECT, either alone or in combination with other imaging methods, preferably radionuclides with an energy of about 30 to about 300 kiloelectronvolts are used. Suitable radionuclide tracers include radionuclides that emit gamma radiation (particularly suitable for SPECT), positrons (particularly suitable for PET), or a combination of gamma radiation and positrons (suitable for either PET or SPECT). In illustrative, non-limiting embodiments, radionuclide tracers have appropriate decay characteristics for optimizing image resolution and quantitative accuracy and/or have appropriate residualization. For example, radionuclide tracers may have a physical half-life (*i.e.,* t½) compatible with the time required for the antibody to achieve optimal specific:non-specific binding ratios. Examples of radionuclide tracers suitable for PET imaging, and suitable for inclusion in a labeled antibody conjugate according to embodiments of the present disclosure, F-18 (t½ about 1.83 hours), Sc-44 (t½ about 3.97 hours), Ti-45 (t½ about 3 hours), Mn-52 (t½ about 5.6 days), Cu-64 (t½ about 12.7 hours), Ga-68 (t½ about 1.13 hours), Br-76 (t½ about 16.2 hours), Y-86 (t½ about 14.7 hours), Zr-89 (t½ about 78.4 hours), 1-124 (t½ about 100.3 hours), or a combination of any two or more thereof. Examples of radionuclide tracers suitable for SPECT imaging include Tc-99m (t½ about 6 h hours), In-111 (t½ about 2.80 days), I-123 (t½ about 13,2 hours), I-131 (t½ about 8 days), Lu-177 (t½ about 6.64 days), Re-186 (t½ about 3.7186 days), or a combination of any two or more thereof.

Preferably, according to the invention a radionuclide is used as part of the label with a physical half-life of between about 1 hour to about 10 days. The selection of the label can be also made based on the fact, whether an antibody or an equivalent binding molecule, *e.g.,* an antibody-fragment has to be labeled, preferably matching the biological half-life of the antibody or antibody-fragment or another equivalent binding molecule. If an antibody is to be labeled, preferably a label with a half-life of at least about 1, 2, 3, 4, 5, 6, 7, 8, 9 or about 10 days, more preferred of about 3-10 days, mostly preferred of about 3-7 or 3-5 days is chosen. If an antibody fragment is to be labeled, a label with a shorter half-life can be selected than for an antibody. Preferably, the label for an antibody-fragment has a half-life of at least 10, 9, 8, 7, 6, 5, 4, 3, 2 or 1 days, more preferred of between about 1 hour to 5 days or between about 1 to 48 hours.

Accordingly, for labelling antibody-fragments for PET a radionuclide with a short physical half-life is chosen, preferably selected from the group consisting of Cu-64, F-18, Ti-45, Br-76, Y-86, Zr-89 or 1-124 or a combination of any two or more thereof. For labelling antibodies for PET preferably a radionuclide with a longer physical half-life is chosen, preferably Zr-89 or I-124. For labelling antibody or antibody fragments for SPECT, preferably a radionucleotide is selected from the group consisting of Tc-99m, In-111, 1-123, 1-131, Lu-177 and Re-186 or a combination of any two or more thereof.

In one embodiment, for PET, shorter-lived radionuclides such as F-18 and Ga-68 are used for labeling binding molecules intended for same-day imaging after injection. In view of their shorter half-life, in one embodiment preferably antibody fragments that have shorter biological half-lives are used with these labels, such as single domain antibodies, scFvs, or diabodies. Nevertheless, in one embodiments also complete antibodies are labeled with these radionucleotides. In one embodiment, longer-lived radionuclides such as Cu-64, Zr-89 and I-124 are preferably used with a complete antibody or a larger antibody fragment, such as the minibody, for optimal imaging contrast 24-48 hours or later post-injection.

The label of the antibody conjugate of the present invention, and potentially further entities, such as a bifunctional chelator or prosthetic group is also referred to as a tracer, if a radionuclide is used as a detection label as radiotracer in the following. According to the invention, bifunctional chelators are preferably used for labelling of antibodies with radiometals, such as Ga-68, Zr-89, and Cu-64 and prosthetic groups are preferably used for labelling with radio-halides, such as F-18, I 123/124/131.

As illustrated in the Examples, if a radionuclide is used as the label or part of the label according to the present invention, the preferred radionuclide is Zr-89. Accordingly, in a preferred embodiment, the label of the antibody conjugate of the present invention is the radionuclide ⁸⁹Zr.

Preferably the detection label is conjugated to the antibody with a bifunctional chelator or prosthetic group. The bifunctional chelator or prosthetic group can be conjugated in any way resulting in a covalent binding to the antibody. Preferably the chelator or prosthetic group is conjugated randomly on amino acid residues, or on glycan regions of the antibody. Preferably, the bifunctional chelator or prosthetic group is conjugated on a lysine or cysteine residue of the antibody. Mostly preferred the bifunctional chelator or prosthetic group is conjugated randomly on lysine residues of the antibody.

According to the invention generally chelators may be used, which have been shown as usable for complexing a radionuclide, a magnetic, paramagnetic or supermagnetic ion complex or particle, to an antibody. Mostly preferred, the bifunctional chelator comprises *p*-SCN-Bn desferoxamine (SCN-Bn-DFO).

In a preferred embodiment of the labeled antibody conjugate of the present invention is the antibody AP-101 or a fragment or biological derivative thereof. AP-101 is described in detail in the international patent application published as WO 2012/080518 A1, wherein the antibody is disclosed as antibody NI-204.12G7. The VH and VL-chain sequences of the antibody and its CDRs are indicated in WO 2012/080518 A1 in Fig. 1B and in Table A below. The constant regions of the antibody are preferably derived from human IgG1. However, the human constant domain in the labeled antibody conjugate of the present invention may be also of a different IgG isotype or of a different allotype, for example to avoid or reduce immunogenicity which can happen as a result of allo-immunization; see, *e.g.,* for review Jefferis and Lefranc, MAbs 1 (2009), 332-338.

The antibody portion of the labeled antibody conjugate of the present invention can be glycosylated or deglycosylated. In one preferred embodiment the antibody is glycosylated, preferably wherein the antibody is produced in a CHO cell. In another embodiment, the antibody is deglycosylated. Preferably, by the deglycosylation the *N*-glycans were removed from the antibody. Deglycosylation can be obtained by different methods according to the invention. Fc-glycans can be deglycosylated, *e.g.,* by an enzymatic treatment with an endoglycosidase acting on complex type N-glycans or the antibody can be produced in a bacterial host such as *E. coli,* wherein no glycosylation has occurred.

The present invention also relates to the labeled antibody conjugate of the present invention disclosed herein for use in a method of diagnosing ALS and monitoring drug therapy and/or progression of ALS in a subject, wherein the method comprises *in vivo* immunoimaging in a subject. Preferably , the imaging of the labeled antibody conjugatein the subject comprises positron emission tomography (PET) imaging, single photon emission computed tomography (SPECT) imaging, magnetic resonance imaging (MRI), magnetic particle imaging (MPI), or PET or SPECT in combination with computed tomography (CT), MRI or MPI.

In a further aspect the present invention generally relates to a drug for use in the treatment of ALS in patient which has been diagnosed for suffering or developing ALS and/or is monitored for efficacy of drug therapy and/or disease progression of ALS by a method as defined herein. Preferably the drug is selected from Riluzole (Rilutek^{®}, Tigultik, Exservan^{™}), Edaravone (Radicava^{®}), Tofersen (BIIB067), AP-101, dextromethorphan HBr and quinidine sulfate (Nuedexta^{®}), Reldesemtiv (CY5031), Arimoclomol, Levosimendan, Fasudil, pyrimethamine, (DaraprimTM), rapamycin, baclofen (Gablofen^{®}, Kemstro^{®}, Lioresal^{®}), diazepam (Diastat^{®}, Valium^{®}), amitriptyline (Elavil^{®}), trihexyphenidyl, Scopoderm^{®} (scopolamine patch), glycopyrrolate (Robinul^{®}), Ravulizumab (also known as BNJ441, ALXN1210, or Ultomiris^{®}) and Eculizumab (also known as Soliris^{®}).

Preferably the labeled antibody conjugate is ⁸⁹Zr-DFO-AP-101 and comprises
(a) AP-101 as the antibody that specifically binds to misfolded SOD1;
(b) p-SCN-Bn desferoxamine (SCN-Bn-DFO) as the bifunctional chelator, and
(c) ⁸⁹Zr as the radionuclide.

In a preferred embodiment, the labeled antibody conjugate of the invention is glycosylated. In another preferred embodiment, the labeled antibody conjugate as defined hereinabove is further characterized by the fact that the AP-101 antibody is deglycosylated. Such a deglycosylated antibody is interchangeably designated as deglycosylated ⁸⁹Zr-DFO-AP-101 or as Degly-⁸⁹Zr-DFO-AP-101. When labeled with Cu-64, such a deglocosylated antibody is interchangeably designated as degylcosylated ⁶⁴Cu-NOTA-AP-101 or as Degly-⁶⁴Cu-NOTA-AP-101.

Furthermore, the present invention provides and relates to an antibody conjugate intermediate, *i.e*., the antibody portion wherein the antibody that specifically binds to misfolded SOD1 comprises a bifunctional chelator, preferably wherein the chelator is *p*-SCN-Bn desferoxamine (SCN-Bn-DFO). Such an antibody conjugate intermediate is particularly useful for preparation of the labeled antibody conjugate of the present invention in that it already comprises the bifunctional chelator conjugated to the antibody and the option that a label can, depending on the intended use, be selected and conjugated thereto. Specific labels which can be conjugated thereto, depending on the imaging technique in which it is intended to use the then conjugate, *i.e*., usable in PET, SPECT, MRI or MPI, PET/CT, PET/MRI, SPECT/CT or SPECT/MRI are listed above.

The present invention further provides a diagnostic composition or kit comprising the labeled antibody conjugate or antibody conjugate intermediate as defined herein (*i.e.,* potentially comprising the deglycosylated or the intermediate forms comprising the chelator), optionally further comprising a detection label, preferably a radionuclide, preferably wherein the radionuclide is Zr-89; and optionally instructions for use in a method of diagnosing amyotrophic lateral sclerosis (ALS) or monitoring drug therapy and/or progression of ALS in a subject as defined in hereinabove.

Furthermore, the present invention also relates to a method of *in vivo* immunoimaging of superoxide dismutase (SOD1) useful as a marker of amyotrophic lateral sclerosis (ALS) or a marker for efficacy of drug therapy of ALS in subject, comprising
(a) administering a labeled-antibody conjugate as defined herein above and below to a subject; and
(b) detecting the presence of the labeled-antibody conjugate in the subject *in vivo* by imaging. The imaging is preferably performed by a method selected from PET, SPECT, MRI and MPI, optical imaging including fluorescence imaging, or from a combination such as PET/CT, SPECT/CT, PET/MRI and SPECT/MRI.

In a further embodiment, the present invention relates to an anti-SOD1 antibody or an equivalent SOD1 binding molecule, for example an antibody fragment as defined further below, for use in targeting misfolded and/or aggregated SOD1 in bone and/or joints of a subject. Herein, under a bone and/or joint related disease a disease is understood which impairs the structure and/or function of bones and/or joints in a subject. Preferably, the antibody or equivalent binding molecule is capable of binding to misfolded and/or aggregated SOD1. Preferably the anti-SOD1 antibody or equivalent SOD1 binding molecule is the antibody AP-101, a SOD1-binding fragment thereof or a labeled antibody conjugate as described herein. Preferably the subject is a mammal, more preferably the subject is a human. In a preferred embodiment the disease is ALS. Furthermore, the present invention also relates to ⁸⁹Zr or ⁸⁹Zr-DFO for use in immunoimaging of misfolded and/or aggregated SOD1 in a subject, preferably for use in the labeled antibody conjugate as disclosed herein and/or for use in early diagnostic of ALS and/or in the monitoring of therapeutic interventions of ALS. Furthermore, in one embodiment a pharmaceutical composition is provided, comprising the anti-SOD1 antibody or an equivalent binding molecule, the labeled antibody conjugate or equivalent SOD1 binding labeled molecule and a pharmaceutically acceptable carrier for use in targeting misfolded and/or aggregated SOD1 in bone and/or joints of a target.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, the exemplary methods and materials are described below. The disclosure content of all publications, patent applications, patents, and other references mentioned herein are incorporated by reference in their entirety. The materials, methods, and examples are illustrative only and not intended to be limiting.

Other features and advantages of the invention will be apparent from the following detailed description and from the claims.

For the avoidance of any doubt it is emphasized that the expressions "in some embodiments", "in a certain embodiment", "in some aspects", "in certain aspects", "in a certain aspect", "in a further aspect", "in certain instances", "in some instances", "in a further embodiment", "in one embodiment" and the like are used and meant such that any of the embodiments described therein are to be read with a mind to combine each of the features of those embodiments and that the disclosure has to be treated in the same way as if the combination of the features of those embodiments would be spelled out in one embodiment. The same is true for any combination of embodiments and features of the appended claims and illustrated in the Examples, which are also intended to be combined with features from corresponding embodiments disclosed in the description, wherein only for the sake of consistency and conciseness the embodiments are characterized by dependencies while in fact each embodiment and combination of features, which could be construed due to the (multiple) dependencies must be seen to be literally disclosed and not considered as a selection among different choices.

### BRIEF DESCRIPTION OF THE DRAWINGS

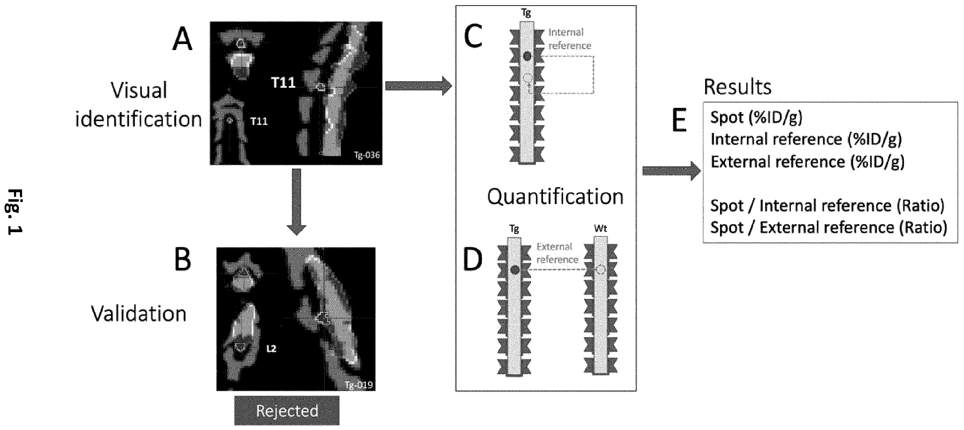

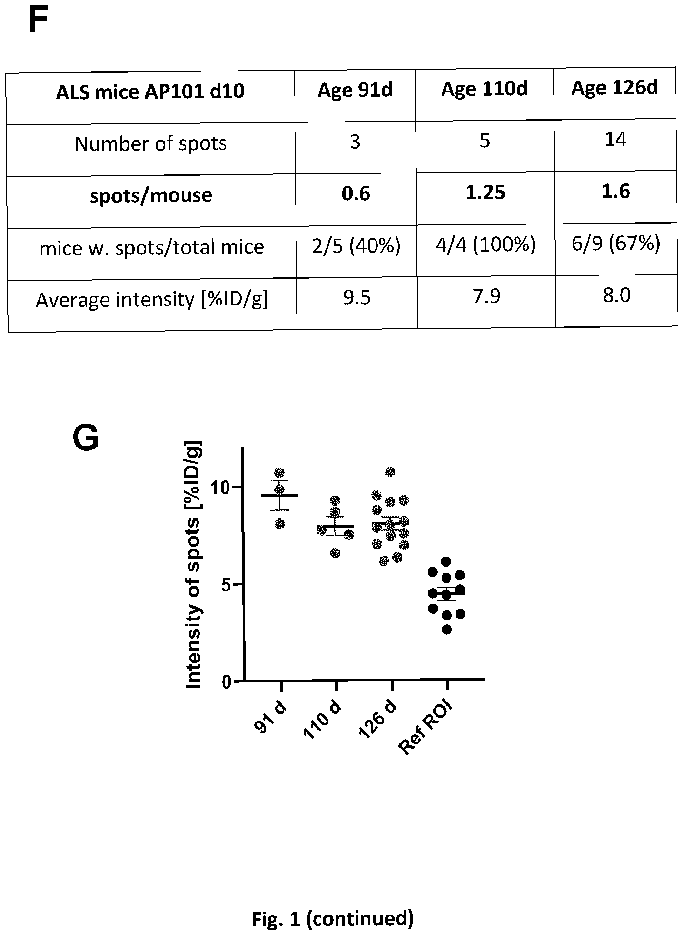

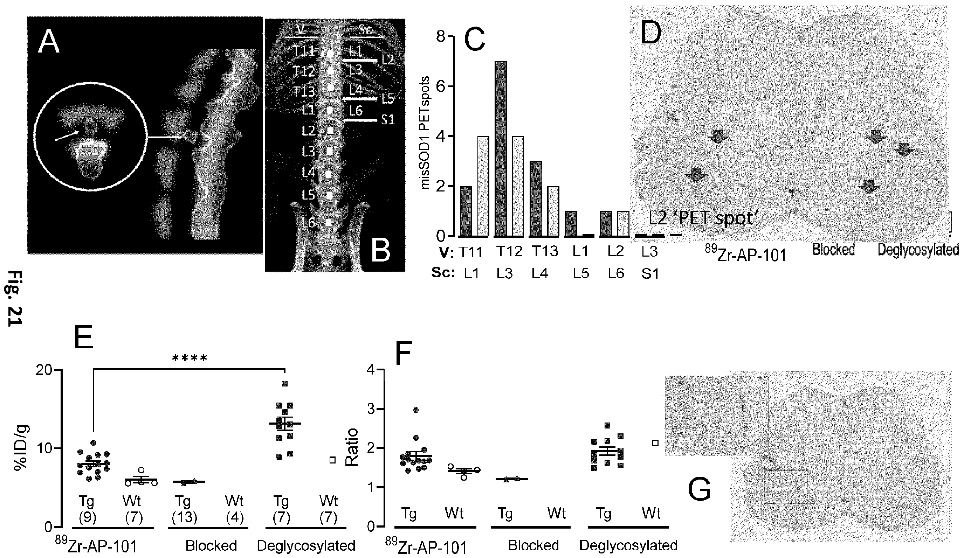

- **Fig. 1:**: Steps in mSOD1 aggregate identification and processing. **(A)** The spots are visually identified on the PET images and a region of interest (ROI) including the whole spot is drawn. **(B)** Spots which are close to the area of spilling from radiotracer in the anterior vertebra bone are not retained for analysis. **(C)** Within the same mouse, a similar ROI is traced on an area of the spinal cord devoid of mSOD1 to obtain an internal reference. **(D)** An external reference is obtained from the spinal cord of the control mice either by drawing a discrete ROI at the level corresponding to the Tg mSOD1 spot or by tracing a ROI including all the control mice spinal cord (profile). **(E)** The amount of radiotracer in mSOD1 spots in unit of %ID/g is reported along the other observed parameters. **(F)** Overview table about the spots counted in the mice depending on the age of the animals. **(G)** Visualization of the spot intensity in comparison to the animals' age. Older animals show more spots/animal.

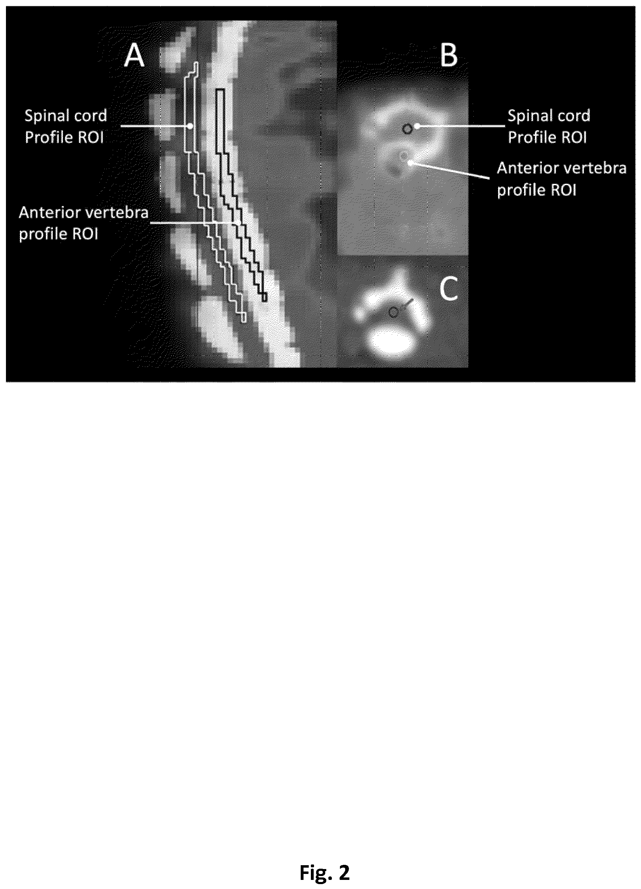

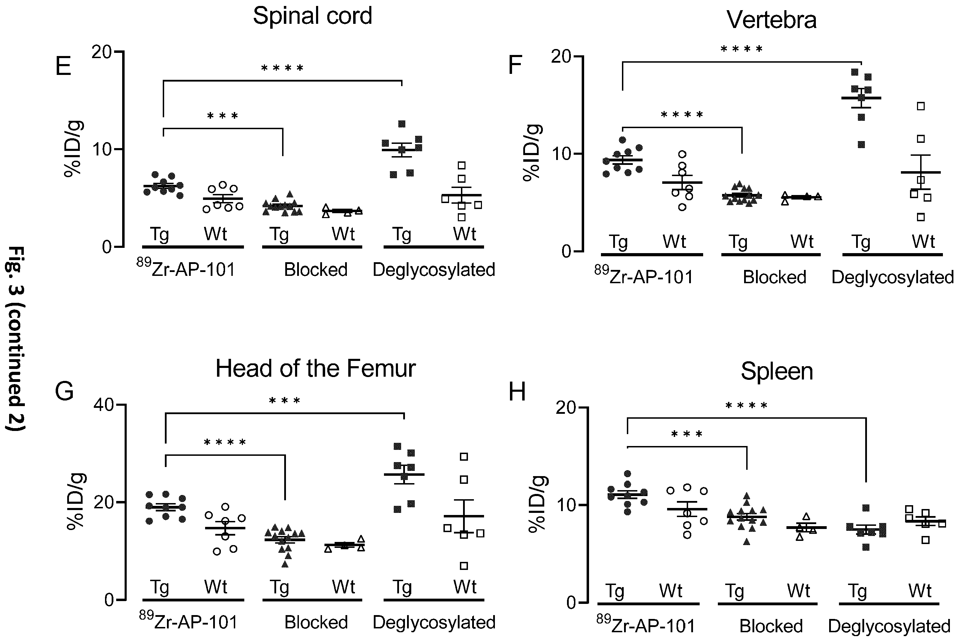

- **Fig. 2:**: Pictures from SOD1 G93A transgenic ALS mice **(A)** Sagittal PET images showing profile ROI traced on the spinal cord and anterior vertebra bone area. **(B)** Transaxial PET image showing the ROI traced on the spinal cord and anterior vertebra. **(C)** Transaxial CT image showing the ROI traced on the spinal cord.

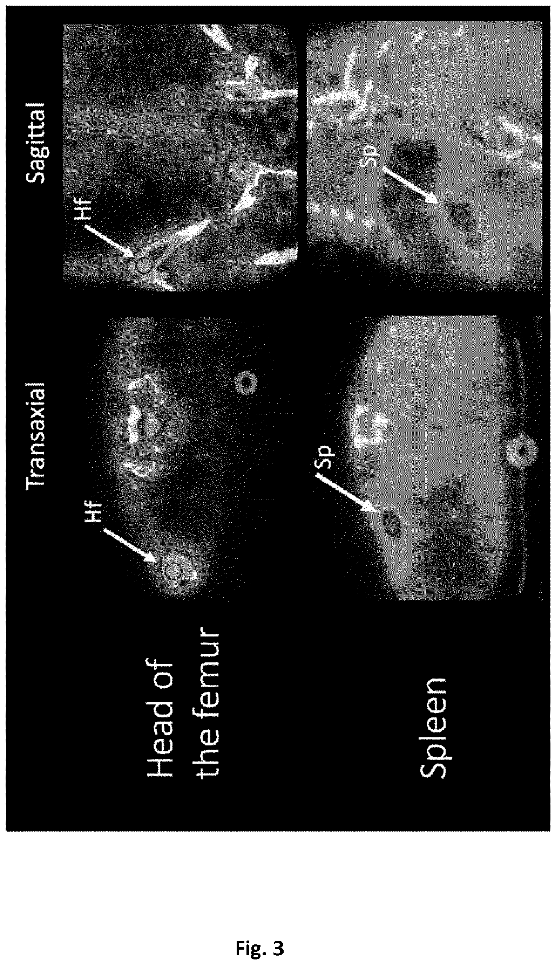

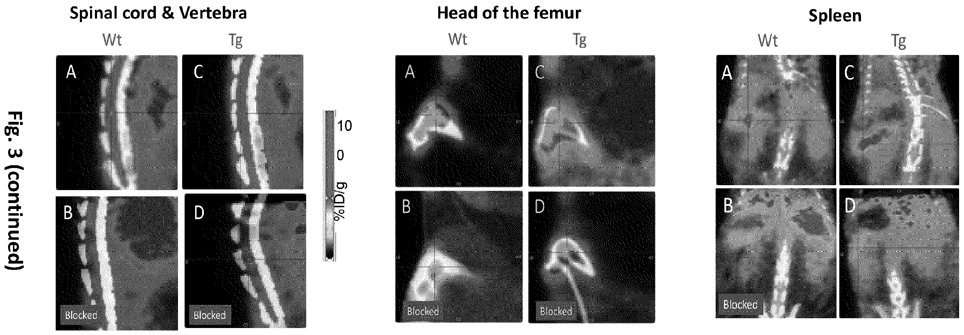

- **Fig. 3**: PET images taken at day-10 following iv administration of ⁸⁹Zr-DFO-AP-101 to 4 months old SOD1 G93A transgenic (Tg) mice, showing the presence of radiotracer in the head of the femur (Top) and in the spleen (Bottom). The arrows point to ROI selected on the head of the femur and the spleen to quantify their radiotracer content. The signal within the spinal cord increased over the time from day 3 to day 10 (not shown).
- **Fig. 3 continued:**: PET images showing the uptake of ⁸⁹Zr-DFO-AP-101 in bone (ventral vertebra, head of the femur) and in the spleen in absence **[(A)** Wt and **(C)** Tg] and presence **[(B)** Wt and **(D)** Tg] of co-injected AP-101 (100 mg/kg) at day 7. Blocking with AP-101 reduced the uptakes of ⁸⁹Zr-DFO-AP-101 in these tissues. The presence of ⁸⁹Zr-DFO-AP-101 in the vertebra bone at time could be intense enough to spill-in the spinal cord making the quantification of the amount of radiotracer in the mSOD-1 spots unreliable. To reduce this effect all images were corrected for spill in and recovery coefficient using a well-established mathematical models as described in Émilie Gaudin et al., Phys. Med. Biol. 66 (2021) 065019, which content is incorporated hereby in its entirety. The uptake of ⁸⁹Zr-DFO-AP-101 in bone and spleen was found to be specific as it was blocked by co-injection of unlabeled AP-101. AP-101 would not only bind to mSOD1 but also to Fc receptor, to reduce Fc binding, Deglycosylated ⁸⁹Zr-DFO AP-101 was synthesized and evaluated. Large effect of blocking in wildtype mice show large contribution of non-misfolded SOD1 signal. Fig. 3 continued 2: In the scattergrams **(E-H)** quantification of PET signal (%ID/g; individual mice with mean value) is provided in the ROI of the spinal cord (E), vertebra bone **(F),** head of the femur **(G)** and spleen **(H)** in SOD1 G93A transgenic and Wildtype mice after injection of ⁸⁹Zr-DFO-α-miSOD1 along with blocking (100 mg/kg unlabeled α-miSOD1) and deglycosylated ⁸⁹Zr-DFO-α-miSOD1. ^{∗∗∗} p<0.001, **** p<0.0001 (One Way ANOVA, Dunnett's multiple comparison test).

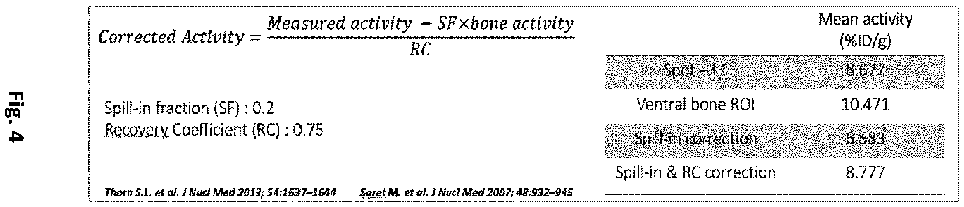

- **Fig. 4**: Radioactivity measured within the spinal cord was corrected from Spill-in of ⁸⁹Zr-tracer present in the ventral vertebra bone and from the partial volume effect using the recovery coefficient RC.

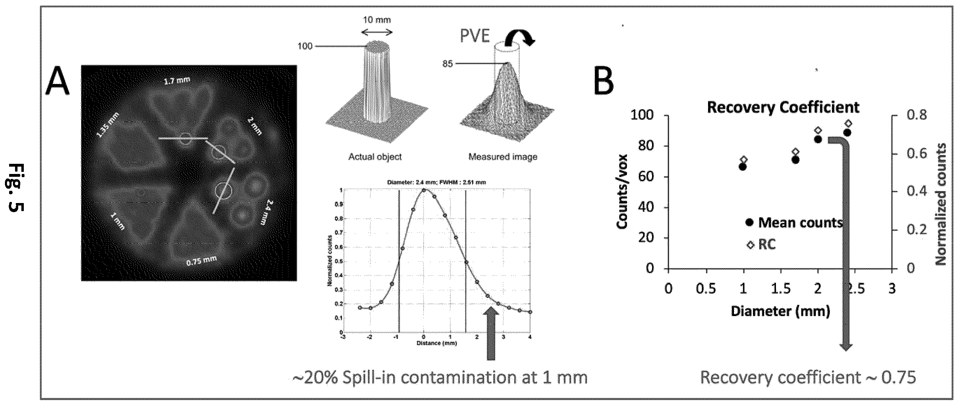

- **Fig. 5**: Partial volume effect (PVE) correction using Ultra-Micro Hot Spot phantom. **(A)** Transverse view of a 0.3 mm slice obtained from the Ultra-Micro hot spot phantom and line profiles across the hot spots at the positions indicated by the yellow lines. **(B)** Mean activity over the hot spots (left y-axis) and recovery coefficients obtained by dividing the hot spot mean activity by the largest hot spot max activity (right y-axis) (Gaudin *et al.* 2021).

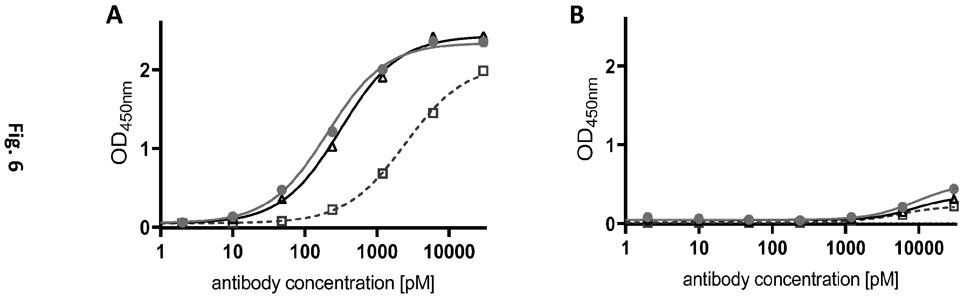

- **Fig. 6**: Binding of ^{nat}Zr-DFO-AP-101 to A) denatured SOD1 and B) native SOD1. In a direct enzyme-linked immunosorbent assay (ELISA) non-modified α-miSOD1 (solid grey line with closed circles), DFO labeled ^{nat}Zr-DFO-AP-101 (solid black line with open triangles) and deglycosylated ^{nat}Zr-DFO-AP-101 (dashed line with open squares) bound with high affinity to denatured human SOD1 (A) with EC₅₀ values of 0.2, 0.3 and 2.4 nM respectively, while showing only minimal binding to the physiological SOD1 dimers (B).

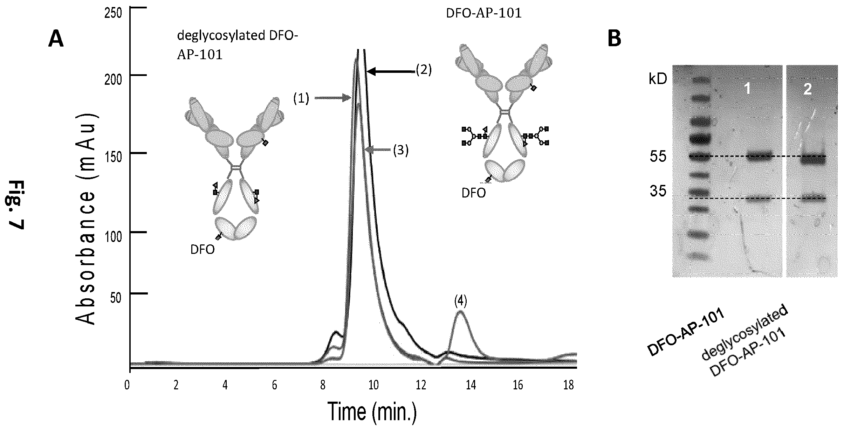

- **Fig. 7**: Characterization of Degly-DFO-AP-101. Characterization and comparison of binding properties of DFO-conjugated, deglycosylated to non-modified AP-101. (A) HPLC tracing of purified deglycosylated DFO-AP-101 (1), DFO-α-miSOD1 (2) and deglycosylated DFO-AP-101 before purification (3) showing the presence of free glycans (4) after 2h incubation with ENDO-S2. (B) SDS-PAGE/Comassie of reduced AP-101 (1) and deglycosylated α-miSOD1 with ENDO-S2 after 2h incubation (2)

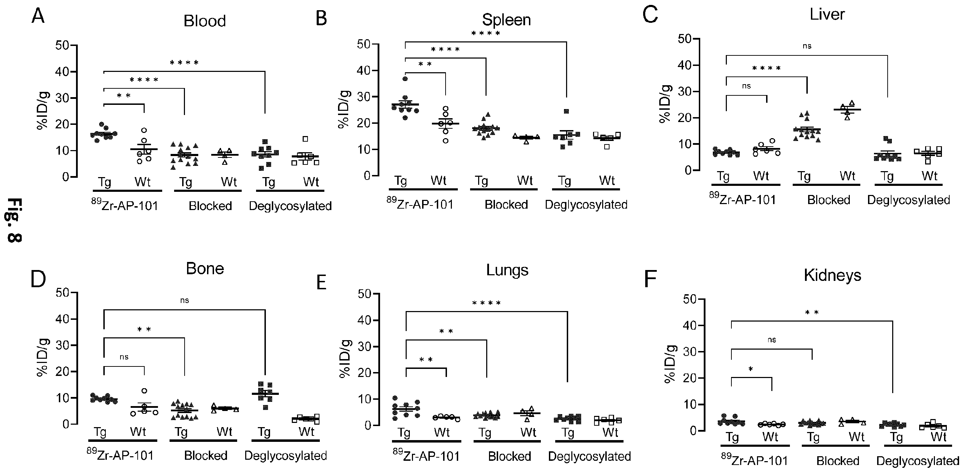

- **Fig. 8**: Residual radiotracer content in non-target organs ex vivo after perfusion of the mice. The residual radioactivity (expressed as percent of the injected dose per gram of tissues; %ID/g) of main non-target tissue 10 days after injection of ⁸⁹Zr-DFO-AP-101, after blocking with 100 mg/kg unlabeled α-miSOD1 (Blocked) or degly ⁸⁹Zr-DFO-AP-101 (Deglycosylated) for SOD1 G93A transgenic (Tg; filled symbols) and wildtype (Wt; open symbols) ) mice measured in total blood (A) or after perfusion of the mice in spleen (B), liver (C), tibia bone (D), lungs (E) and kidneys (F). Mean %ID/g ±SEM; ns not significant, ** p<0.01, *** p<0.001, ****p<0.0001 (One Way ANOVA; Dunnett's post-hoc)

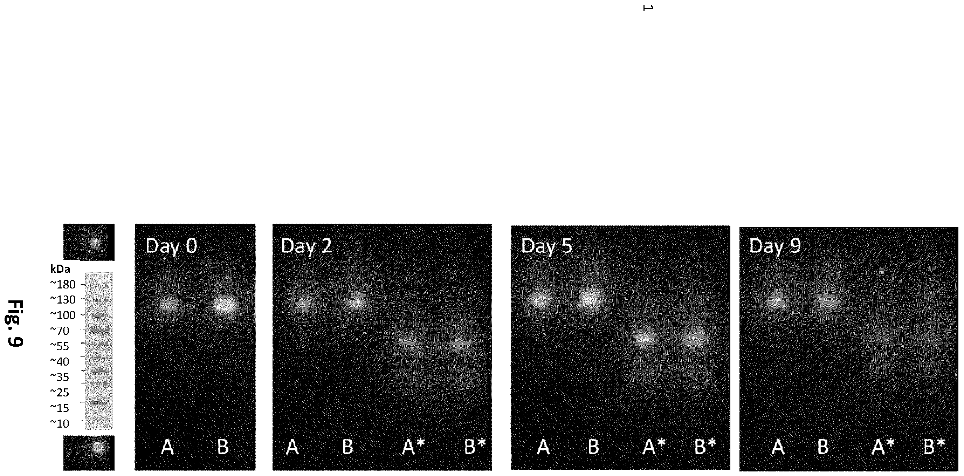

- **Fig. 9**: SDS-PAGE of Degly-⁸⁹Zr-DFO-AP-101 formulated in PBS alone **(A)** or in presence of GA **(B).** *Monitored under reducing conditions.

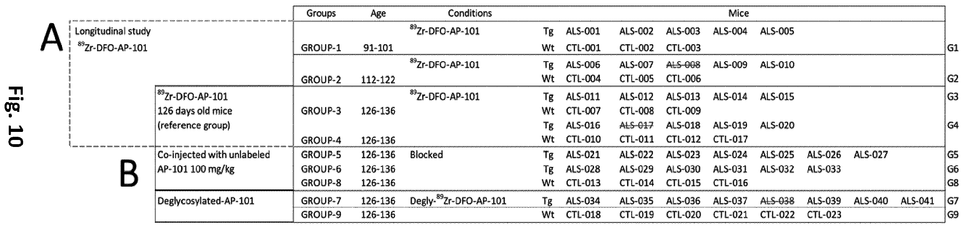

- **Fig. 10**: Illustration of experimental groups/conditions described herein: distribution of the transgenic (Tg or ALS) and wild type (Wt or CTL) mice within the different experimental groups. The study was performed in Tg [B6.Cg - Tg(SOD1^{∗}G93A)1Gur/J)] mice expressing the misfolded form of SOD1 and their control Wt [C56BL/6J] mice. The study was undertaken to select the optimal mice age and time post administration of ⁸⁹Zr-DFO-AP-101 to carry PET Imaging. Following this study, it was decided to use mice older than 126 days and to image at days 7 and 10 post administration of the radiotracer. The feasibility of using ⁸⁹Zr-DFO-AP-101 for the detection of mSOD1 aggregated in the spinal cord was evaluated by blocking with unlabeled AP-101 to establish the specificity of ⁸⁹Zr-DFO-AP-101 for mSOD1. Deglycosylation was also explored as a mean to evaluate the impact of Fc binding of ⁸⁹Zr-DFO-AP-101. **(A)** Previously completed longitudinal study aimed at selecting the best age and day post administration of the radiotracer for PET imaging. **(B)** Current study assessing the feasibility of using ⁸⁹Zr-DFO-AP-101 for PET imaging of misfolded SOD1 (mSOD1).

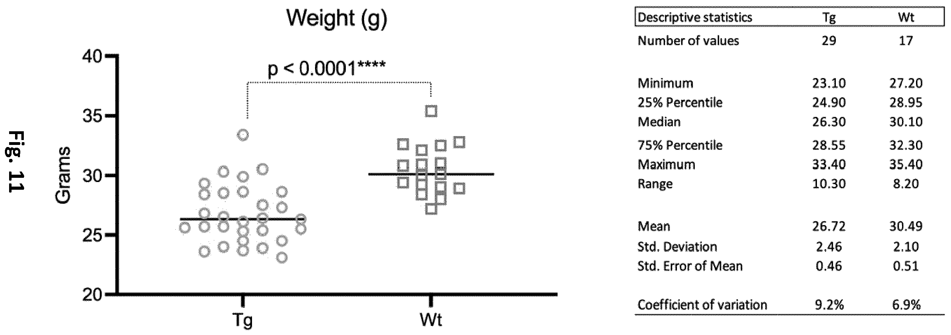

- **Fig. 11**: Scattergram comparing the weight of 126 days old Tg and Wt mice and descriptive statistics. The Tg and Wt mice weights were compared using an unpaired Two-tails Student t test.

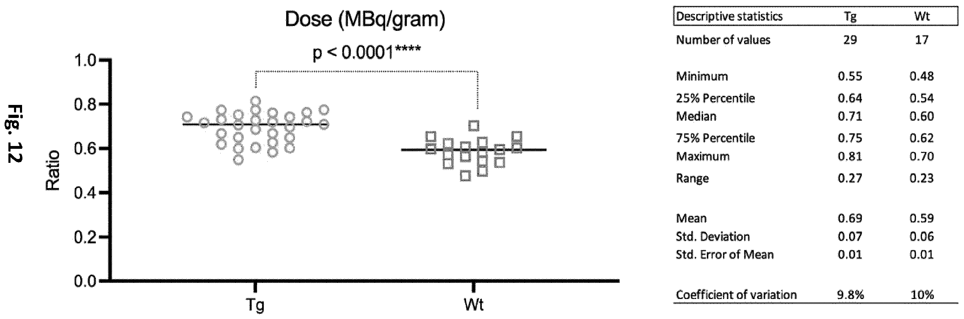

- **Fig. 12**: Scattergram comparing the dose per gram of radiotracer administrated to 126 days old Tg and Wt mice and descriptive statistics. The Tg and Wt mice received doses were compared using an unpaired Two-tails Student t test.
- **Fig: 13**: Distribution of mSOD1 spots within the mice spinal cord. The spots detected with either ⁸⁹Zr-DFO-AP-101 and its deglycosylated analog are represented in the left MIB PET/CT image. The false (+) spot, which includes control (Wt) mice and blocked mice (Tg and Wt) presenting valid spots are also included in the figure. Data from the table are also visualized in Fig. 21 I-J.
- **Fig: 14**: Bar-graph comparing the frequency of mSOD1 spot detection (% mice with spots), average intensity of the detected mSOD1 spots and the mSOD1 spot to internal reference ratio. The error bar stands for the standard deviation of the mean (sdm). The number on top of the bars represents the number of mice in the group. The error bars represent the sample standard deviation of the mean. The "*p*" values were obtained using a Two-tails Student-t test.
- **Fig: 15**: The internal reference values measured on the spinal cord of Tg mice receiving the deglycosylated analog were significantly higher than those measured in the Tg mice receiving ⁸⁹Zr-DFO-AP-101 (Two-tails Student-t test). This higher background value will contribute to increasing the %ID/g value of the mSOD1 spots from the Degly-⁸⁹Zr-DFO-AP-101 mice.
- **Fig: 16**: Whole body maximal intensity projection PET/CT images showing the biodistribution of ⁸⁹Zr- AP- **(A, D)** in 4 months old SOD1 G93A transgenic **(Tg, A, B, C)** and control wildtype **(D, E, F)** mice along with blocking **(B, E;** 100 mg/kg unlabeled AP-101 and deglycosylation conditions **(C, F)** in liver, spleen, spinal cord, ventral vertebra bone and head of the femur. **(G)** annotation of positive areas.
- **Fig: 17**: Scattergram comparing the spinal cord uptake (A), the vertebra ventral bone uptake (B) and the spinal cord to vertebra ventral bone uptake ratio (C) of ⁸⁹Zr-DFO-AP-101 to that of the blocked conditions and Degly-⁸⁹Zr-DFO-AP-101. The %ID values were obtained from profile ROI traced on the spinal cord. For each condition, the %ID/g values measured in the Tg (orange; first scattergram in each figure) and Wt (blue; second scattergram in each figure) mice were compared using a Two-tail Student-t test, the "p" values are given at the bottom of the graph. The %ID/g values for the Tg mice receiving ⁸⁹Zr-DFO-AP-101 were compared to those of the blocked and deglycosylated groups using a One-way ANOVA and a Dunnett's multiple comparison test to compare the values obtained with ⁸⁹Zr-DFO-AP-101 with the blocked and deglycosylated conditions, (dotted lines and "p" values).
- **Fig. 18**: Correlation between the %ID/g values measured by PET imaging or by biodistribution. Except for the spinal cord where the tissue content in radiotracer was low. There was a significant correlation between the %ID/g values measured by PET imaging or biodistribution.
- **Fig. 19**: Compilation of mSOD1 identification data at day-10 post administration of the radiotracer and structure of ⁸⁹Zr-DFO-AP-101 and of its deglycosylated analog.

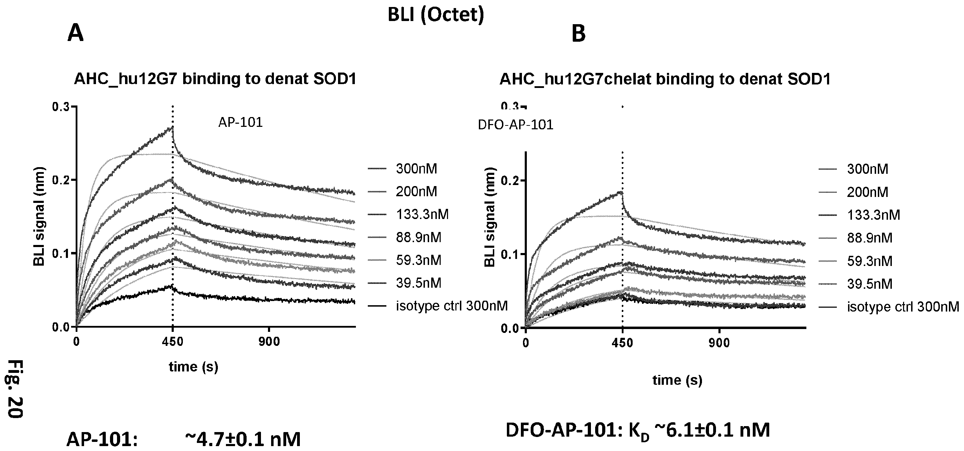

- **Fig. 20**: ⁸⁹Zr-DFO-AP-101 (B) binds in a similar manner to soluble misfolded SOD1 compared to non-modified AP-101 (A) in Bio-Layer Interferometry (BLI). Absolute signal and K_{D} is slightly reduced for the Chelator-modified AP-101 suggesting some minor changes.
- **Fig. 21**: Localization and quantification of PET positive misSOD1 aggregates using ⁸⁹Zr-DFO-AP-101 and direct binding of DFO-AP-101 to misSOD1 aggregates using immunohistochemistry on spinal cord sections. **(A)** Transaxial (left, circle) and sagittal (right) PET/CT-image of a PET positive spot identified within the spinal cord (arrow). **(B)** Dorsal view of the CT image showing the vertebral column of a SOD1 G93A transgenic mice and the vertebra classification (V, left, T11-L6) and the corresponding spinal cord region applied (Sc, right, L1-S1 based on Harrison, 2013). **(C)** Bar graph showing the number of misSOD1 aggregates identified on the PET images at each vertebra level for Immuno-PET with ⁸⁹Zr-DFO-AP-1011 (dark grey bar) and its deglycosylated analog (light grey bar). **(D)** Percent of SOD1 G93A transgenic (Tg, dark grey) and Wildtype (Wt, light grey) mice presenting valid spots for ⁸⁹Zr-DFO-AP-101, ⁸⁹Zr-DFO-AP-101 co-injected with 100 mg/kg of AP-101 (Blocked) and deglycosylated ⁸⁹Zr-DFO-AP-101 respectively. The total number of spots per total number of mice imaged is presented above each column. **(E)** Quantification of radiotracer in the misSOD1 aggregates (%ID/g) for transgenic (black) and Wt mice (open symbol) with ⁸⁹Zr-DFO-AP-101, blocked or deglycosylated analogs. Mean±SEM of all spots and the number of mice imaged for each group is indicated. (**** p<0.0001, One Way ANOVA, Dunnett's multiple comparison test for SOD1 G93A mice). **(F)** Ratio of the misSOD1 aggregate radiotracer content to internal background (internal reference ROI traced on area of the spinal cord of the same mouse devoid of a misSOD1 spots) for SOD1 G93A transgenic (black symbol) and wildtype mice (open samples) with ⁸⁹Zr-DFO-AP-101, blocked and deglycosylated conditions. Mean ± SEM of all spots. **(G)** Transaxial histological section through the spinal cord of a SOD1 G93A transgenic mouse showing the misSOD1 pathology labelling using immunohistochemistry with α-miSOD1 at a location where positive PET-spots has been identified (see insert) after injection of ⁸⁹Zr-DFO-AP-101.
- **Fig. 22**: Misfolded SOD1 detection in the bone of SOD1 G93A and in SOD1 G37R (Fig. 22 continued) transgenic mice with AP-101 and commercial misSOD1 antibody (B8H10 from MediMaps). While no immunoreactivity could by detected with the isotype control antibody (1nM isotype Ctrl row)) or in wildtype mice, misSOD1 agglomerates (exemplarily marked with black arrows) can be observed in bone and cartilage tissue of vertebrae, and joint area of two different SOD1 transgenic mice, G93A and G37R.

### DETAILED DESCRIPTION OF THE INVENTION

The present invention relates to the embodiments as characterized in the claims, disclosed in the description and illustrated in the Examples and Figures further below.

In particular, the present invention relates to antibodies and antibody conjugates, respectively, which are useful in the diagnosis and monitoring treatment/progression of diseases due to or associated with misfolded SOD1, in particular neurogenerative diseases such as amyotrophic lateral sclerosis (ALS).

The present invention is based on the concept of using an anti-SOD1 antibody that selectively binds to misfolded and/or aggregated SOD1 for targeting such SOD1 species in human tissue of ALS patients and visualize target engagement by a detectable label *in vivo.* It was hoped that by means of such labeled anti-SOD1 antibody conjugate the severity and progress of the disease could be determined qualitatively and quantitatively.

Tracers that can facilitate ALS diagnosis, aid in prognosis, and measure drug pharmacodynamics are needed to accelerate therapeutic development for patients with ALS and identify patients at an early stage of the disease. Positron emission tomography (PET) imaging with an appropriate radiotracer has great potential for ALS given that it would permit visualization of central nervous system (CNS) pathology in individuals living with the disease.

Recently, a recombinant human monoclonal antibody, with selective binding to human misfolded SOD1 (mSOD1) has been investigated, designated AP-101, which has been originally disclosed in international application WO 2012/080518 A1 and further characterized in Maier et al., Sci. Transl. Med. 10 (2018)(470) doi: 10.1126/scitranslmed.aah3924. Besides its disease-modifying properties, recent experiments suggest a high specificity of this anti-SOD1 antibody for mSOD1.

The AP-101 antibody has been shown to identify misfolded SOD1 in post-mortem spinal cord tissue of both familial and sporadic ALS patients regardless of their SOD1 genotype (Maier *et al.* 2018). To further confirm this hypothesis the present inventors developed a proprietary sensitive assay for the detection of mSOD1 in biological fluids, specifically cerebral spinal fluid (CSF), from ALS patients (ALS-Detect ELISA). According to preliminary data from a controlled collection dataset generated to support the assay development, it was possible to determine that misfolded SOD1 was present in the CSF of a large majority of SOD1 mutant FALS patients tested. These results suggest that screening the CSF of patients with known disease is useful for a confirmation of ALS but suggests that the invasive assay may not yet have sufficient sensitivity to identify disease onset in all patients.

Direct imaging of the spine with a positron emission tomography (PET) and single photon emission computed tomography (SPECT), and also Magnetic resonance imaging (MRI), Magnetic particle imaging (MPI) and optical imaging have the potential as a diagnostic tool to detect upper motor neuron (UMN) pathology in ALS patients without UMN symptoms, as a prognostic tracer that might help stratify patients for clinical trials, and as a pharmacodynamic tracer that measures the biological effect of investigational drugs in the brain and spinal cord.

PET is a powerful imaging technique that can follow the distribution of picomolar concentrations of radiopharmaceuticals. PET imaging is non-invasive, functional and quantitative and has proven to be a highly valuable technology platform for the study of other neurodegenerative diseases. It also represents a powerful technology to tackle proof-of-efficacy studies for drug development. The availability of sensitive PET imaging scanners for preclinical research and the introduction of PET-CT for preclinical and clinical use could be used for an early and certain ALS diagnosis if a specific radiotracer was developed. Similarly, SPECT; MRI, MPI and optical imaging including fluorescence imaging, or combinations thereof and/or with PET *(e.g.,* PET/CT, PET/MRI, PET/MPI) are used to selectively visualize molecular targets for which a specific tracer could be used, *e.g.,* for detection of misfolded SOD1 and diagnosis of ALS and/or monitoring of the disease progression/treatment.

For investigating this approach, a labeled-antibody conjugate, DFO-AP-101 was prepared by conjugating AP-101 antibody with p-SCN-Bn desferoxamine (SCN-Bn-DFO) chelator randomly on lysine residues and the Degly-DFO-AP-101 (deglycosylated antibody-chelator) was prepared *via* an enzymatic treatment of DFO-AP-101 with an endoglycosidase from *Streptococcus pyogenes* that specifically hydrolyzes glycans at the Fc glycosylation site of IgG (see Example 1 and corresponding Preparation sections in Materials and Methods below). Both antibodies were analyzed *via* SDS-PAGE (see Figs. 7 and SEC-HPLC prior labeling with ⁸⁹Zr, which has been selected as an exemplary radionuclide in view of the intended use of PET for immunoimaging. The specific activity and the number of DFO chelators per antibody were determined to fully characterize the PET tracers (see Example 1 and Table 1). An automated process for the production of ⁸⁹Zr-DFO-AP-101 conjugates was developed using a cassette-based module (see Example 1 and corresponding Preparation sections in Materials and Methods below). Stability studies were performed to establish the formulation and the shelf-life of the radiotracers (see Example 2 and section Stability studies in Materials and Methods). A longitudinal imaging study was performed to identify the optimal mice age and time post administration of ⁸⁹Zr-DFO-AP-101 for the detection misfolded SOD1 aggregation in symptomatic and pre-symptomatic transgenic (Tg, [B6.Cg - Tg(SOD1^{∗}G93A)1Gur/J)]) mice expressing misfolded SOD1, leading to the formation of m-SOD1 aggregates. A 3D region of interest (ROI) was drawn to include the whole mSOD1 spots and the radiotracer content was expressed in terms of percent of injected dose per gram equivalent (%ID/g). The weight in gram of mSOD1 aggregates was estimated from the ROI volume assuming a density of 1g/mL. Control imaging experiments were done with ⁸⁹Zr-DFO-AP-101 and Degly-⁸⁹Zr-DFO-AP-101 in Wt mice. The mice were imaged using a small animal PET/CT scanner following caudal vein administration of either ⁸⁹Zr-DFO-AP-101 or Degly-⁸⁹Zr-DFO-AP-101 (see Examples 3-6, and *e.g.,* Figs. 2, 3, 13, 16). A group of mice receiving ⁸⁹Zr-DFO-AP-101 were co-injected with unlabeled AP-101 to assess target specificity (see Fig. 3 (continued 2) and Figs. 10, 13 and 19). After the last imaging session (Day-10) the mice were euthanized, the tissues were sampled, and their radiotracer content was measured in a gamma counter.

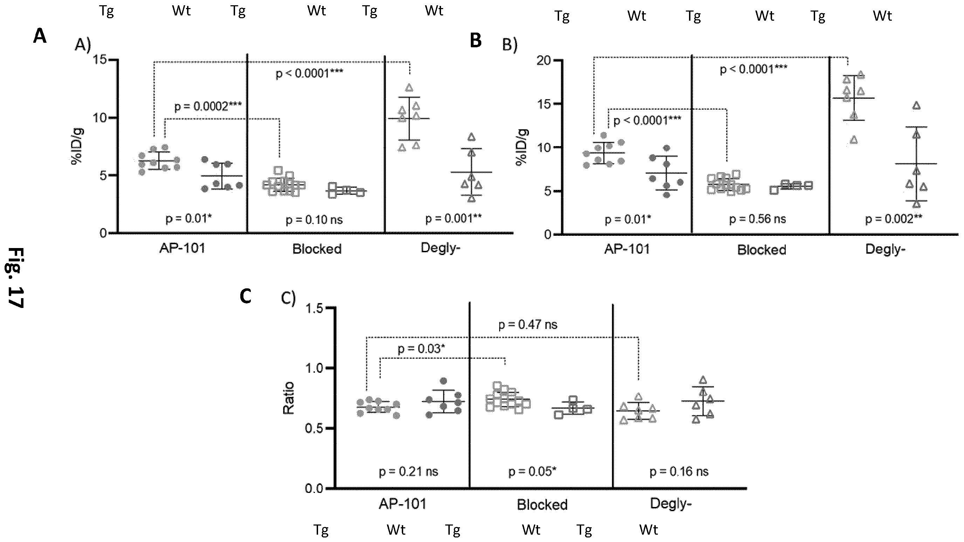

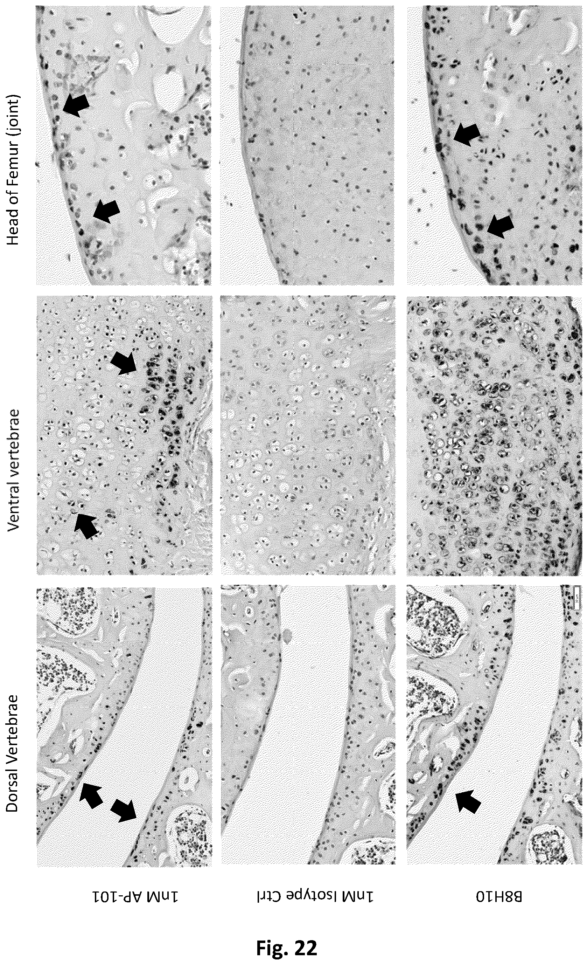

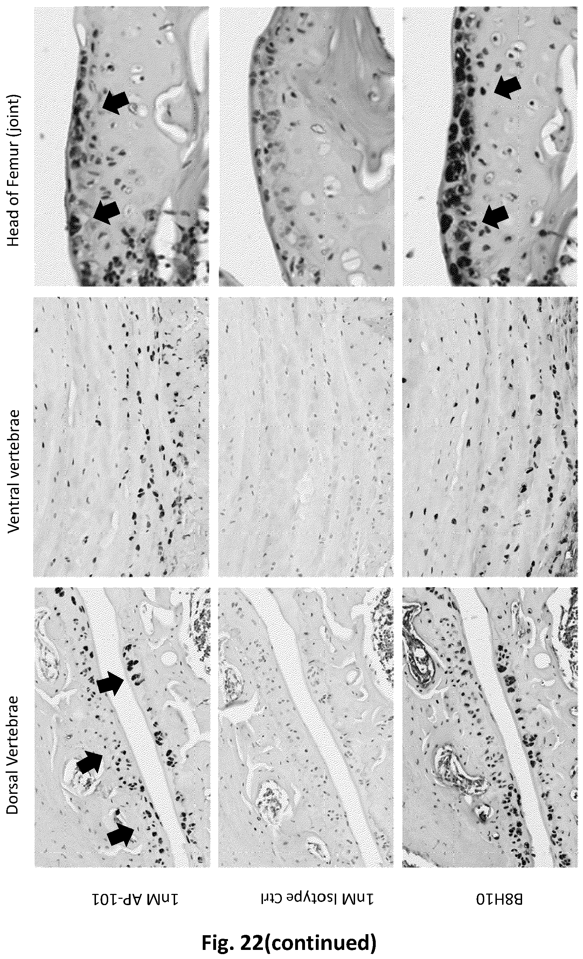

Both radiotracers investigated herein ⁸⁹Zr-DFO-AP-101 and Degly-⁸⁹Zr-DFO-AP-101 were able to engage mSOD1 aggregates in the spinal cord of Tg mice and imaging was optimal in 126-day-old Tg mice and at day 7 and 10 post tracer administration (Figs. 2-4, 13). The uptake of ⁸⁹Zr-DFO-AP-101 was specific, as it was being blocked when co-injected with AP-101 (Fig. 3 continued and Figs. 10, 13 and 19). Degly-⁸⁹Zr-DFO-AP-101 showed an improved uptake in mSOD1 aggregate but this tracer was also more concentrated in the anterior vertebra bone of Tg mice causing radiation spilling into the spinal cord resulting in similar aggregate to spinal cord tissue ratios for both radiotracers (Figs. 3, 4). A low background in spinal cord area of Wt mice was observed for Degly-⁸⁹Zr-DFO-AP-101 resulting in a better contrast between Wt and Tg mice. Spleen and liver uptakes were lower for Degly-⁸⁹Zr-DFO-AP-101 compared to ⁸⁹Zr-DFO-AP-101 (Example 4, Fig. 17 and Fig. 3 (continued 2)), but the bone uptake of Tg mice (vertebra and head of the femur) was higher suggesting the presence of mSOD1 in bone and/or bone marrow of Tg mice (Example 5, Fig. 3 and Fig. 22).

Although ⁸⁹Zr-DFO-AP-101 and Degly-⁸⁹Zr-DFO-AP-101 showed similar mSOD1 to spinal cord ratio, Degly-⁸⁹Zr-DFO-AP-101 seems to have the most potential for clinical applications. The rationale for this choice is that the Degly-⁸⁹Zr-DFO-AP-101 was more avidly concentrated in mSOD1 aggregates offering a high contrast between Wt and Tg mice.

In view of the results provided by the experiments described herein and summarized above, the present invention generally relates to a labeled-antibody conjugate for use in a method of diagnosing amyotrophic lateral sclerosis (ALS) and/or monitoring drug therapy and/or progression of ALS in a subject. The method preferably uses *in vivo* immunoimaging of misfolded SOD1 (mSOD1) in the subject. For this aim the labeled-antibody conjugate preferably comprises an antibody that specifically binds to misfolded and/or aggregated SOD1, and a detection label conjugated to the antibody or antibody-fragment. Preferably, the antibody (with and without label) specifically binds misfolded and/or aggregated mammalian SOD1 such as mouse, rat or human misfolded and/or aggregated SOD1, in particular preferred it binds human misfolded and/or aggregated SOD1.

Preferably the label is either directly or indirectly covalently bound to the antibody. If the label is indirectly conjugated to the antibody, preferably a bifunctional chelator or linker is used, which is then covalently bound to both the label and the antibody.

As mentioned, the labeled-antibody conjugate interchangeably also named as "conjugate" herein (independently of the fact whether the label is directly, or according to the preferred embodiment, indirectly bound to the antibody, *e.g.,* also independently of the fact whether the label is conjugated or complexed) according to the present invention is for use in methods preferably comprising *in vivo* immunoimaging of mSOD1, by which the conjugate allows, by making mSOD1 visible, to determine whether mSOD1 is present in a subject and if present, in addition to its presence also visualization of mSOD1 distribution in the organs and/or tissues of the subject. Presence of mSOD1, and in particular of mSOD1 comprising aggregates in particular in the spinal cord is, as indicated above, a hallmark for ALS both in patients and in transgenic (Tg) animal models. In this connection, any method known in the art for *in vivo* immunoimaging can be used. Preferably, however, the particular immunoimaging method is selected from the group comprising or consisting of PET, SPECT, MRI, MPI and optical imaging including fluorescence imaging, and combinations thereof or combinations with CT, such as PET/CT, SPECT/CT and PET/MRI, SPECT/MRI. In particular preferred the imaging or the immunoimaging method respectively comprises or is PET, particularly preferred the imaging or the immunoimaging method respectively comprises or is a combination of PET and CT as exemplarily shown in Figs. 2, 3 and 21A.

Depending on the imaging method intended to be used for diagnosing ALS or monitoring drug therapy and/or progression of ALS in a subject, the particular label is selected for the labeled-antibody conjugate.

Preferably, the label to be included in the labeled antibody conjugate or labeled antibody fragment conjugate is selected from: Fluorine-18 (F-18), Titanium-45 (Ti-45), Manganese-52 (Mn-52), Iron-52 (Fe-52), Kalium-43 (K-43), Scandium-43 (Sc-43), Scandium-44 (Sc-44), Cobalt-57 (Co-57), Copper-60 (Cu-60), Copper-61 (Cu-61), Copper-62 (Cu-62), Copper-64 (Cu-64), Copper-67 (Cu-67), Gallium-67 (Ga-67), Gallium-68 (Ga-68), Bromine-76 (Br-76), Bromine-77 (Br-77), Krypton-81m (Kr-81m), Rubidium-81 (Rb-81), Yttrium-86 (Y-86), Strontium-87m (Sr-87m), Zirconium-89 (Zr-89 or ⁸⁹Zr), Technetium-99m (Tc-99m), Indium-111 (In-111), Indium-113m (In-113m), Antimony-117 (Sb-117), Iodine-123 (I-123), Iodine-125 (I-125), Caesium-127 (Cs-127), Caesium-129 (Cs-129), Iodine-131 (1-131), Iodine-132 (I-132), Lutetium-177 (Lu-177), Rhenium-186 (Re-186), Quicksilver-197 (Hg-197), Rhenium-188 (Re-188), Lead-203 (Pg-203), Bismuth-206 (Bi-206), Actinium-225 (Ac-225), Radium-225 (Ra-225) and a combination of any two or more thereof.

More preferred, the label is selected from the group comprising or consisting of:
(a) for use in PET from: Fluorine-18 (F-18), Manganese-52 (Mn-52), Copper-64 (Cu-64), Gallium-67 (Ga-67), Gallium-68 (Ga-68), Antimony-117 (Sb-117), Scandium-43 (Sc-43), Scandium-44 (Sc-44), Titanium-45 (Ti-45), Bromium-76 (Br-76), Rubidium-82 (Rb-82), Yttrium-86 (Y-86), Iodine-124 (I-124) and Zirconium-89 (Zr-89 or ⁸⁹Zr);
(b) for use in SPECT from: Indium-111 (In-111), Technetium-99m (Tc-99m), Iodine-123 (I-123), Iodine-131 (1-131), Lutetium-177 and Rhenium-186 (Re-186);
(c) for use in MRI and MPI: magnetic, paramagnetic or superparamagnetic ion complexes or particles, preferably containing Gaddolinum³⁺ (Gd³⁺), Manganese²⁺ (Mn²⁺); Copper ²⁺ (Cu²⁺); iron oxide (Fe₃O₄) contrast agents; and
(d) for use in optical imaging including fluorescent imaging from: fluorescein, Cy2, Cy3, Cy3B, Cy3.5, Cy5, Cy5.5, Cy7, Texas Red@ (Molecular Probes, Inc., Eugene, OR), AlexaFluor^{®} (Molecular Probes, Inc., Eugene), AlexaFluor 350, AlexaFluor 405, AlexaFluor 430, AlexaFluor 488, AlexaFluor 500, AlexaFluor 532, AlexaFluor 546, AlexaFluor 568, AlexaFluor 594, AlexaFluor 610, AlexaFluor 633, AlexaFluor 647, AlexaFluor 660, AlexaFluor 680, AlexaFluor 700, AlexaFluor 750, BODIPY FL, BODIPY R6G, BODIPY TMR, BOPDIPY 530/550, BODIPY 558/568, BODIPY 564/570, BODIPY 576/589, BODIPY 581/591, BODIPY 630/650, BODIPY 650/665, Rhodamine Green^{™}-X (Molecular Probes, Inc.), Rhodamine Red^{™}-X (Molecular Probes, Inc.), Rhodamine 6G, TMR, TAMRA^{™} (Applied Biosystems).

Preferably, the labeled antibody-conjugate for use according to the present invention is provided, wherein the detection label is a radionuclide. The label may comprise one or more radionuclides. More preferably, the one or more radionuclide(s) is (are) selected from the radionuclides listed under item (a) or (b) in the preceding paragraph. Mostly preferred, the radionuclide is selected from the radionuclides listed under item (a) in the preceding paragraph. In particular preferred, the conjugate of a label and an antibody as defined herein, is provided, wherein the radionuclide is Zr-89.

The presence and position of the labeled antibody-conjugate or equivalent binding moleculein the subject is detected in the subject by the selected imaging method preferably in real time. Preferably the imaging method is PET or a combination of PET and CT.

Since *in vivo* imaging methods, such as PET, require administration of the labeled conjugate to a subject, as a first step, before performing the imaging method, preferably a different screening method is used, which is less invasive. Preferably, an *in vitro* method is used in this connection, in which marker are detected in a body fluid sample obtained from a patient, which are indicative of ALS. Particularly preferred a mSOD1 screening method is used as described in another application of the present inventors, WO 2021/185961 A1, the content of which is hereby incorporated herein in its entirety by reference.

Accordingly, preferably, before or as confirmation of the immunoimaging method according to the present invention, screening for mSOD1 in a sample of a patient's body fluid is performed (pre-screen). The sample to be analyzed with the assay of the present invention may be any body fluid suspected to contain pathologically mSOD1, for example a blood, CSF, or urine sample. In a preferred embodiment, the sample is whole blood lysate or CSF, preferably CSF.

The screening method applies an ELISA immunoassay comprising contacting the body fluid with two different anti-SOD1 antibodies. In detail, the prescreen is performed by the use of methods as described in Examples 1 to 2 on pages 23 to 25 or as described in Example 3 on pages 26 to 28 of WO 2021/185961 A1, which methods are hereby expressly incorporated herein by reference.

Preferably the detection label is conjugated to the antibody specifically binding to misfolded and/or aggregated SOD1 with a bifunctional chelator or linker, such as a prosthetic group. The bifunctional chelator or prosthetic group can be conjugated in any way resulting in a covalent binding to the antibody. Preferably the chelator or prosthetic group is conjugated randomly on amino acid residues, or to a glycan region of the antibody. Preferably, the amino acid residues on which the chelator or prosthetic group is randomly conjugated are lysine or cysteine residues. Mostly preferred the bifunctional chelator or prosthetic group is conjugated randomly on one or more lysine residues of the antibody.

A detection label is conjugated directly, or indirectly, to an anti-SOD1 antibody to produce a labeled antibody conjugate according to the present invention.

According to embodiments, broadly described, direct conjugation of the detection label to an anti-SOD1 (in particular anti-mSOD1) antibody according to the present disclosure includes reaction of a functional group of the detection label, or a compound containing the detection label, with a corresponding functional group of the antibody, or antibody fragment, such as a terminal amino group, a terminal carboxyl group or a functional group of an amino acid side chain, thereby covalently bonding the detection label with the antibody, or antibody fragment.

According to the present invention, however, indirect conjugation of the detection label to an antibody, or antibody fragment is preferred. Such indirect conjugation includes reaction of a functional group of a linker, prosthetic group, or chelator with a corresponding functional group of the antibody, or -antibody fragment, such as a terminal amino group, a terminal carboxyl group or a functional group of an amino acid side chain, thereby covalently bonding the prosthetic group, or chelator with the antibody, or antibody fragment, and binding of the detection label or a compound comprising the detection label to the linker or chelator.

Functional groups and exemplary conjugation reactions are known in the art as exemplified in G. T. Hermanson, Bioconjugate Techniques, 2nd Edition, Academic Press, 2008.

For indirect conjugation of a detection label to an antibody or antibody fragment, a suitable prosthetic group, or chelator, preferably a bifunctional chelator can be used, wherein the prosthetic group, or chelator is bound to both the antibody, or antibody fragment, and the detection label. Preferably, the prosthetic group, or chelator is bifunctional and therefore functional to bind to both the antibody, or antibody fragment, and the detection label, producing a labeled antibody conjugate.

Prosthetic groups, chelators, and methods of use thereof for conjugation of a detection label to an antibody, or antibody fragment, are well-known in the art, see for example, Shan S. Wong et al., Chemistry of Protein and Nucleic Acid Cross-Linking and Conjugation, Second Edition, CRC Press, 2011. Preferred bifunctional chelators to be used in accordance with the present invention are listed further below.

In embodiments, the labeled-antibody conjugates include at least one detection label conjugated to the antibody, or antibody fragment, wherein the at least one detection label includes a radionuclide. In embodiments, the labeled-antibody conjugates include at least one detection label, is a fluorophore. In embodiments, the at least one detection label is a magnetic/paramagnetic or supermagnetic particle. In embodiments, the labeled-antibody conjugates include at least one detection label, a metal-containing particle. Particular particles include, but are not limited to, carbon-, gold-, or iron-containing nanoparticles.

In embodiments, labeled-antibody conjugates are not limited with respect to the number of labels included. In embodiments, labeled-antibody conjugates include 1, 2, 3, 4, 5, 6, 7, 8, 9, 10 labels, or more. Where multiple labels are included, they may be the same or different.

According to the present invention generally chelators may be used, which have been shown as usable for complexing a radionuclide, a magnetic, paramagnetic or supermagnetic ion complex or particle to an antibody. Preferably the chelator used in accordance with the present invention for complexing labels, preferably radionuclides, to the antibody comprises or is selected from the group consisting of SCN-Bn DFO*, SCN-Bn-HOPO, SCN-Bn-4HMSA, NOTA (2-[4,7-bis(carboxymethyl)-1,4,7-triazonan-1-yl]acetic acid) and its derivatives; DOTA (2-[4,7,10-tris(carboxymethyl)-1,4,7,10-tetrazacyclododec-1-yl]acetic acid) and its derivatives; methylhydroxamates derived from triaza- and tetraazamacrocycles (NOTHA₂ and DOTHA₂) and its derivatives; 1,4,7-triazacyclononane-1-glutaric acid-4,7-diacetic acid (NODAGA) and its derivatives; diethylenetriaminepentaacetate (DTPA) and its derivatives; 1,4,7,10-tetraazadodecane-1 ,4,7-triacetate (D03A) and its derivatives; 3,6,9,15-tetraazabicyclo[9.3.1]pentadeca-1 (1 5),11,13-triene-3,6,9-triacetic acid) (PCTA) and its derivatives; 1,4,7,10- tetraazacyclotridecanetetraacetic acid (TRITA) and its derivatives; 1,4,8,11- tetraazacyclotetradecane-1,4,8,11-tetraacetic acid (TETA) and its derivatives; 1,4,7,10-tetraazadodecanetetramethylacetate (DOTMA) and its derivatives; 1,4,7,10-tetraazadodecane-1,4,7-trimethylacetate (D03MA) and its derivatives; N,N',N",N‴-tetraphosphonatomethyl-1,4,7,10-tetraazacyclododecane (DOTP) and its derivatives; 1,4,7,10-tetraazacyclododecane-1,4,7,10-tetrakis(methylene methylphosphonic acid) (DOTMP) and its derivatives; 1,4,7,10-tetraazacyclododecane-1,4,7,10-tetrakis(methylene phenylphosphonic acid) (DOTPP) and its derivatives; N,N'-ethylenedi-L-cysteine and its derivatives; N1,N1'-(butane-1,4-diyl)bis(N4-hydroxy-N1-(3-(4-(hydroxy(methyl)amino)-4-oxobutanamido)propyl)-N4-methylsuccinamide) (4HSM) and its derivatives; and *p*-SCN-Bn desferoxamine (SCN-Bn-DFO; also known as *p*-SCN-Bn deferoxamine).

In a preferred embodiment, if the antibody is intended to be labeled with Zr-89, generally ⁸⁹Zr-chelators can be used such as desferrichrome (DFC), HMSA, four 1-hydroxypyridin-2-one groups appended to a linear tetraamine (HOPO), L1-4, L5, FSC derivatives, TAFC, FOXE, CP256, YM103, DFO-star (DFO*), oxoDFO* and *p*-SCN-Bn desferoxamine.

Other chelators can be used in combination with other radiometals: NOTA or its derivatives; methylhydroxamates derived from triaza- and tetraazamacrocycles (NOTHA2 and DOTHA2); 1,4,7-triazacyclononane-1-glutaric acid-4,7-diacetic acid (NODAGA) or its derivatives; diethylenetriaminepentaacetate (DTPA) or its derivatives; 1,4,7, 10-tetraazadodecanetetraacetate (DOTA) and its derivatives; 1,4,7,10- tetraazadodecane-1,4,7-triacetate (D03A) and its derivatives; 3,6,9,15-tetraazabicyclo[9.3.1]pentadeca-1(15),11,13-triene-3,6,9-triacetic acid) (PCTA) or its derivatives; 1,4,7,10- tetraazacyclotridecanetetraacetic acid (TRITA) and its derivatives; 1,4,8,11- tetraazacyclotetradecane-1,4,8,1 1-tetraacetic acid (TETA) and its derivatives; 1,4,7,10-tetraazadodecanetetramethylacetate (DOTMA) and its derivatives; 1,4,7,10-tetraazadodecane-1,4,7-trimethylacetate (D03MA) and its derivatives; N,N',N",N‴-tetraphosphonatomethyl-1,4,7,10-tetraazacyclododecane (DOTP) and its derivatives; 1,4,7, 10-tetraazacyclododecane- 1,4,7, 10-tetrakis(methylene methylphosphonic acid) (DOTMP) and its derivatives; 1,4,7,10- tetraazacyclododecane-1,4,7,10-tetrakis(methylene phenylphosphonic acid) (DOTPP) and its derivatives; or N,N'-ethylenedi-L-cysteine or its derivatives.

Mostly preferred, the bifunctional chelator comprises *p*-SCN-Bn desferoxamine (SCN-Bn-DFO; also known as *p*-SCN-Bn deferoxamine).

According to the invention, different prosthetic groups can be used for 18F (or F-18)-labeling. Preferred prosthetic groups for that use comprise N-succinimidyl 4 [18F]-fluorobenzoate ([18F]-SFB), 4-azidophenacyl-[18F]-fluoride ([18F]-APF), and 1-(3-(2-[18F]fluoropyridin-3-yloxy)propyl)pyrrole-2,5-dione ([18F]-FpyMe).

The antibody for use in a method of diagnosing ALS or monitoring drug therapy and/or progression of ALS in a subject as specified herein, can in principle be any anti-SOD1 antibody that selectively binds to misfolded and/or aggregated forms of SOD1, preferably preferentially or exclusively over the physiological form of SOD1.

In principle, the antibody which is part of the labeled antibody conjugate of the present invention may be in any format recognizing misfolded and/or aggregated SOD1 comprising, for example chimeric antibody, single-chain antibody, Fab-fragment, bi-specific antibody (a diabody or minibody), fusion antibody, a unibody or an analog of any one of those. Corresponding methods for producing such variants are known to the person skilled in the art and are described, *e.g.,* in Harlow and Lane "Antibodies, A Laboratory Manual", CSH Press, Cold Spring Harbor (1988) First edition; Second edition by Edward A. Greenfield, Dana-Farber Cancer Institute © 2014, ISBN 978-1-936113-81-1. For example, Fab and F(ab')₂ fragments may be produced recombinantly or by proteolytic cleavage of immunoglobulin molecules, using enzymes such as papain (to produce Fab fragments) or pepsin (to produce F(ab')₂ fragments). F(ab')₂ fragments contain the variable region, the light chain constant region and the CH1 domain of the heavy chain.

In one embodiment, the antibody for use according to the present invention may thus be provided in a format selected from the group consisting of a single chain Fv fragment (scFv), an F(ab') fragment, an F(ab) fragment, and an F(ab')₂ fragment, an Fd, an Fv, a single-chain antibody, a single domain antibody (sdAb, also known as VHH Abs or nanobodies) and a disulfide-linked Fv (sdFv) and/or which is a chimeric murine-human or a humanized antibody. Preferably, the anti-SOD1 antibody employed comprises a human constant domain. The term "equivalent binding molecule" as used herein is intended to refer to the formats indicated as alternatives to an antibody in this and in the preceding paragraph.

The antibody AP-101, which corresponds to antibody NI-204.12G7 as characterized in WO 2012/080518 A1 and in Maier et al., Sci. Transl. Med. 10. (2018) doi: 10.1126/scitranslmed.aah3924 (the disclosure content of which is explicitly incorporated herein by reference in its entirety) is particularly preferred and is a fully human IgG1m3 allotype antibody with selective high affinity binding to misfolded SOD1 protein.

Thus, in one embodiment, the labeled antibody for use in accordance with the present invention, *i.e*., in particular for use in a diagnosing method as defined above, is an anti-SOD1 antibody recognizing an epitope comprising the amino acid sequence 73-GGPKDEERHVG-83 (SEQ ID NO: 11).

In a particular preferred embodiment the antibody for use in accordance with the present invention is AP-101 and derived from human antibody NI-204.12G7 and characterized by comprising in its variable region, *i.e.* binding domain the complementarity determining regions (CDRs) of the variable heavy (V_{H}) and variable light (V_{L}) chain having the amino acid sequences depicted in Fig. 1B of WO 2012/080518 A1, and as replicated in Table A, below, or an equivalent antibody wherein one or more of the CDRs may differ in their amino acid sequence from those set forth in Fig. 1B of WO 2012/080518 A1 and in Table A below by one, two, three or even more amino acids in case of CDR2 and CDR3, and wherein the antibody displays substantially the same or identical immunological characteristics of anti-SOD1 antibody NI-204.12G7 illustrated in the Examples of WO 2012/080518 A1. The positions of the CDRs are shown in Fig. 1B and explained in the Figure legend to Fig. 1 in WO 2012/080518 A1, and also shown in Table A below. The corresponding nucleotide sequences are set forth in Table II at page 54 of WO 2012/080518 A1 and listed in Table A below.

**Table A:** Amino acid and nucleotide sequences of the variable region, *i.e*., heavy chain and kappallambda light chain of human antibodies of the AP-101 antibody. Framework (FR) and complementarity determining regions (CDRs) are indicated with the CDRs being underlined in the amino acid sequences. The heavy chain joining region (JH) and light chain joining region (JK) are indicated as well. Due to the cloning strategy the amino acid sequence at the N-terminus of the heavy chain and light chain may potentially contain primer-induced alterations in FR1, which however do not substantially affect the biological activity of the antibody. In order to provide a consensus human antibody, the nucleotide and amino acid sequences of the original clone were aligned with and tuned in accordance with the pertinent human germ line variable region sequences in the database; see, *e.g.,* Vbase (http://vbase.mrc-cpe.cam.ac.uk/) hosted by the MRC Centre for Protein Engineering (Cambridge, UK). Those amino acids, which are considered to potentially deviate from the consensus germ line sequence due to the PCR primer and thus have been replaced in the amino acid sequence, are indicated in bold.

| | |
|---|---|
| AP-101-V_{H} | GAGGTGCAGCTGGTGGAGTCTGGGGCTGAGGTGAAGAAGCCTGGGGCCTCAGT GACACTGTCCTGCAAGGCATCTGGATACACCTTCACCGCCTACTATATACACT GGGTGCGACAGGCCCGAGAACAAGGGCTTGAGTGGATGGGCGTAATCAACCCT AGTACTGGAACCACATTTTACGCACAGAACTTCCCGGACAGAGTCTCCGTGAC CAGGGACACGTCCACGAGTACAGTCTTCATGGAGCTGCACAACCTGAAATCTG AGGACACGGCCGTATATTACTGTGCGAGAGCAATCAGTGAGCATGGTTCAGGG AGTTATTCACCTTATTACTGGGGCCAGGGCACCCTGGTCACCGTCTCCTCG SEQ ID NO:12 |
| DNA sequence | |
| AP-101-V_{H} | FR1-----------------------------CDR1-FR2---------- QVQLVQSGAEVKKPGASVTLSCKASGYTFTAYYIHWVRQAREQGLEWMG E    E CDR2-------------FR2------------------------------ VINPSTGTTFYAQNFPDRVSVTRDTSTSTVFMELHNLKSEDTAVYYCAR CDR3----------JH--------- AISEHGSGSYSPYYWGQGTLVTVSS     SEQ ID NO: 1 (version with Q and Q) and SEQ ID NO:2 (version with E and E) |
| amino acid sequence | |
| AP-101-V_{L} | TCCTATGTGCTGACTCAGCCACCCTCGGTGTCAGTGTCCCTAGGACAGATGGC CGCGATCACCTGCTCTGGAGAGGCATTGCCAAAAAAGTATGGTTATTGGTACC AGCAGAAGCCAGGCCAGGTCCCTGTTCTGCTAATTTATAGAGACGTCGAGAGG CCCTCAGGGGTCCCTGACCGATTCTCTGGCTCCAGCTCAGGGACAATGGTCAC ATTGACCATCAGTGGAGTCCAGGCAGAGGACGAGGCTGACTATTACTGTCTCT CAGCAGACAGCAGTGGTACTTGGGTGTTCGGCGGAGGGACCAAGCTGACCGTC CTA     SEQ ID NO:13 |
| DNA sequence | |
| AP-101-V_{L} | FR1--------------------CDR1-------FR2----------- SYELTQPPSVSVSLGQMAAITCSGEALPKKYGYWYQQKPGQVPVLLIY V CDR2---FR3---------------------------CDR3------ RDVERPSGVPDRFSGSSSGTMVTLTISGVQAEDEADYYCLSADSSGTWV JK-------- FGGGTKLTVL      SEQ ID NO:6 (version with E), SEQ ID NO: 7 (version with V at position 3) |
| amino acid sequence | |

In addition, or alternatively, the framework regions or complete V_{H} and/or V_{L} chain are 80% identical to the framework regions depicted in Table A above (and Fig. 1B of WO 2012/080518 Al), preferably 85%, 90%, 95%, 96%, 97%, 98%, 99% or 100% identical to the framework regions and V_{H} and/or V_{L} chain, respectively, depicted in Table A above (and Fig. 1B of WO 2012/080518 A1). Furthermore, cloning and expression of antibody NI-204.B has been performed as described in WO 2012/080518 A1 in the section "Material and methods" at pages 84 to 88 the description of which methods is thus incorporated herein by reference.

In a particular preferred embodiment, the antibody is characterized by the CDRs and/or the V_{H} and/or V_{L} chain depicted in Fig. 1B of WO 2012/080518 A1 and in Table A above.

Thus, the antibody for use as defined herein preferably comprises (besides the detection label conjugated to the antibody as characterized herein):
(i) a variable heavy (VH) chain comprising VH complementary determining regions (CDRs) 1, 2, and 3, and/or a variable light (VL) chain comprising VL CDRs 1, 2, and 3, wherein
   (a) VH-CDR1 comprises the amino acid sequence of SEQ ID NO: 3 or a variant thereof, wherein the variant comprises one or two amino acid substitutions,
   (b) VH-CDR2 comprises the amino acid sequence of SEQ ID NO: 4 or a variant thereof, wherein the variant comprises one or two amino acid substitutions,
   (c) VH-CDR3 comprises the amino acid sequence of SEQ ID NO: 5 or a variant thereof, wherein the variant comprises one or two amino acid substitutions,
   (d) VL-CDR1 comprises the amino acid sequence of SEQ ID NO: 8 or a variant thereof, wherein the variant comprises one or two amino acid substitutions,
   (e) VL-CDR2 comprises the amino acid sequence of SEQ ID NO: 9 or a variant thereof, wherein the variant comprises one or two amino acid substitutions, and
   (f) VL-CDR3 comprises the amino acid sequence of SEQ ID NO: 10 or a variant thereof, wherein the variant comprises one or two amino acid substitutions; and/or
(ii) a VH chain and/or a VL chain, wherein
   (a) the VH chain comprises the amino acid sequence depicted in SEQ ID NO: 1 or 2 or a variant thereof, wherein the variant comprises one or more amino acid substitutions; and
   (b) the VL chain comprises the amino acid sequence depicted in SEQ ID NO: 6 or 7, or a variant thereof, wherein the variant comprises one or more amino acid substitutions;
preferably wherein the VH and VL chain amino acid sequence is at least 90% identical to SEQ ID NO: 1 or 2 and 6 or 7, respectively.

Accordingly, most preferably the anti-SOD1 antibody for use in a method of diagnosing amyotrophic lateral sclerosis (ALS) or monitoring drug therapy and/or progression of ALS in a subject of the present invention is AP-101 or an equivalent binding molecule or an mSOD1-binding fragment derived from human antibody NI-204.12G7 as characterized in WO 2012/080518 A1 and Maier et al., Sci. Transl. Med. 10. (2018) doi: 10.1126/scitranslmed.aah3924.

The five primary classes of immunoglobulins are IgG, IgM, IgA, IgD and IgE. These are distinguished by the type of heavy chain found in the molecule. IgG molecules have heavy chains known as gamma-chains; IgMs have mu-chains; IgAs have alpha-chains; IgEs have epsilon-chains; and IgDs have delta-chains; see for review, *e.g.,* Schroeder et al., J. Allergy Clin. Immunol. 125 (2010), S41-S52. Furthermore, different subclasses exist, wherein the IgAs are further divided into subclasses IgA1 and IgA2, and wherein IgGs are further divided into subclasses IgG1, IgG2, IgG3, and IgG4. Furthermore, two types of light chain, kappa (κ) and lambda (λ) exist.

In principle, the antibody for use in accordance with the present invention may be of any kind of class and subclass, respectively, and may comprise any kind of light chain, as long as the antibody binds to misfolded and preferably aggregated forms of SOD1, and preferably as long as binding specificity towards SOD1 as indicated in the Examples of WO 2012/080518 A1 for antibody NI-204.12G7 remains unaffected in kind. However, preferably complete IgG antibodies are used, wherein the antibody comprises a constant domain. Accordingly, in one embodiment, the immunoglobulin heavy and/or light chain constant domain present in the antibody as used in accordance with the present invention is of the IgG type, the IgM type, the IgA type, the IgD type or the IgE type, preferably of the IgG type. In one embodiment, the immunoglobulin heavy and/or light chain constant domain present in the antibody as used in accordance with the present invention is of the IgA1, IgA1, IgG1, IgG2, IgG3, or IgG4 subclass, preferably of the IgG1, IgG2, IgG3, or IgG4 subclass and most preferably of the IgG1 subclass.

Recombinant expression of complete human IgG1 antibodies with a human or mouse constant domain can be performed substantially as described in the Examples of WO 2012/080518 A1. Preferably, the antibody is a monoclonal antibody or derived from a monoclonal antibody.

There are not only the above-mentioned four subclasses of IgGs but human heavy and light chain genes also exhibit extensive structural polymorphism(s) and, being closely linked, are inherited as a haplotype. Allotypic variants can be immunogenic and provoke antibody responses as a result of allo-immunization. Thus, switching the allotype can be of particular interest to provide non-immunogenic antibody. So far, extensive allotypes (polymorphisms) are known, but focus is put on the serologically defined allotypes. Allotypes of IgG proteins are defined by the expression of unique epitope(s) recognized by unique serologic reagent(s).

Allotypes expressed on the constant region of IgG heavy chain are designated as Gm (Genetic marker) together with the subclass, *e.g.,* G1m, and the allotype number (or letter), *e.g.,* G1m1 [or G1m(a)], G3m5 [or G3m(b1)]. Human immunoglobulin allotypes are listed in Table 1 of Jefferis and Lefrance, mAbs 1 (2009), 1-7 and in Fig. 1A of Irani et al., Molecular Immunology 67 (2015), 171-182, which content is herein incorporated by reference. Accordingly, in one embodiment, the antibody for use in accordance with the present invention is of any one of the following allotypes, but not limited thereto: G1m1, G1m2, G1m3, G1m17, G2m23, G3m21, G3m28, G3m11, G3m5, G3m13, G3m14, G3m10, G3m15, G3m16, G3m6, G3m24, G3m26, G3m27, A2m1, A2m2, A2m3, Em1, Km1, Km2, and Km3, but preferably of G1m2, G1m3, or G1m17, and most preferably of G1m3.

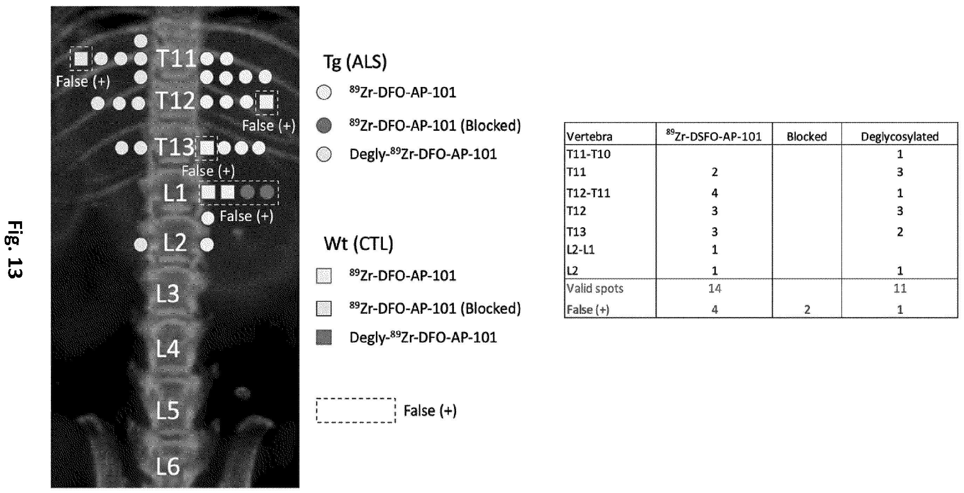

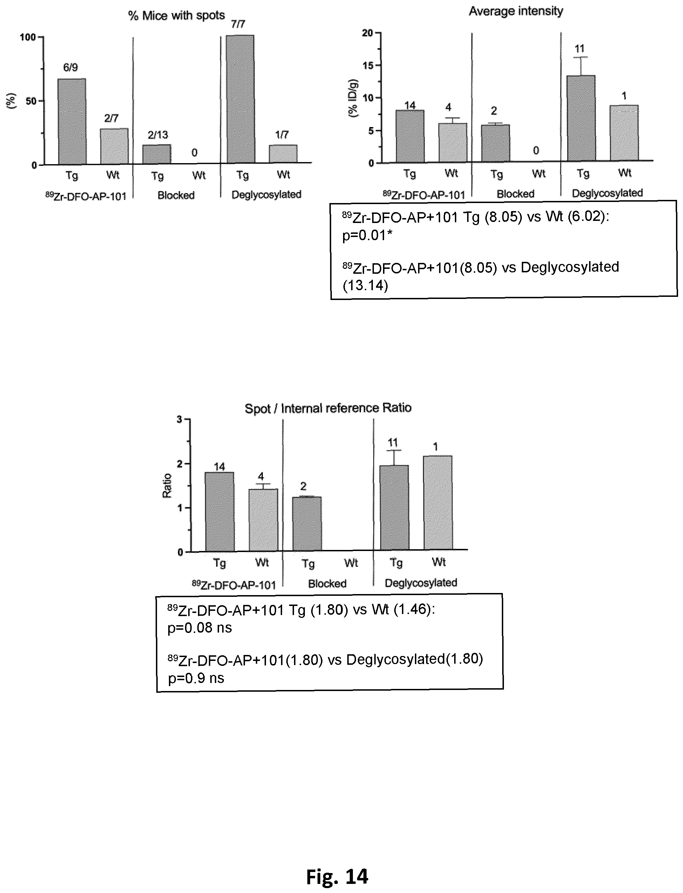

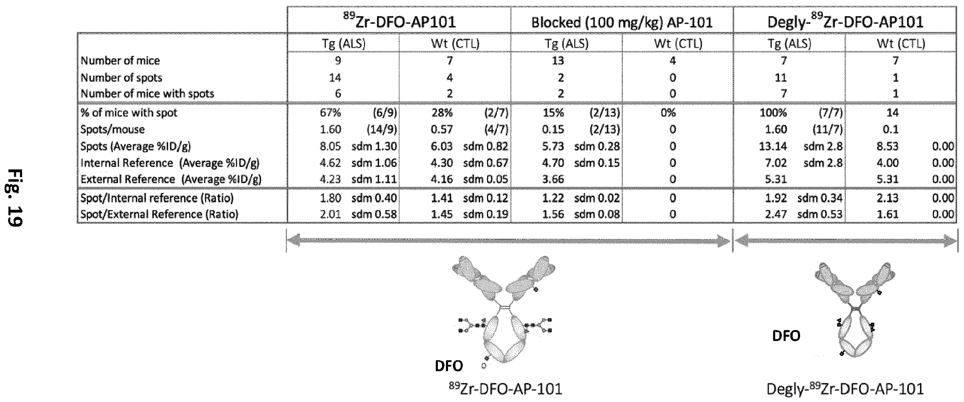

Glycosylation, *i.e*., attachment of glycans, is a common post-translational modification that occurs during the production of antibodies (Wang *et al.* (2020)). As shown in the experiments described in the Examples below, deglycosylation increased the efficacy of the radiotracer to detect mSOD1 aggregates from 67% to 100% (Example 3, Fig. 14). However, both radiotracers (*i.e.,* also the glycosylated) were found to be able to engage spinal cord mSOD1 aggregates, the highest uptake and number of detected mSOD1 aggregates being obtained with Degly-⁸⁹Zr-DFO-AP-101 (Fig. 13, 14). Deglycosylation significantly increased the uptake of the radiotracer in vertebra bone and the head of the femur (Example 5, Fig. 3G) suggesting the presence of mSOD1 in bone or bone marrow and a specific uptake in these tissues. Moreover, lower spinal cord and vertebra background was seen in Wt mice that received Degly-⁸⁹Zr-DFO-AP-101 resulting in better contrast between Wt and Tg mice with this ⁸⁹Zr-based AP-101 radiotracer (Example 4, Fig. 17 and Fig. 3 (continued 2)). For these reasons, both radiotracers are suitable, however, Degly-⁸⁹Zr-DFO-AP-101 seems to be the most suitable radiotracer for clinical application. Therefore, the antibody for use as specified herein is provided in two forms, in the glycosylated or in the deglycosylated form (Fig. 19 (scheme)).

Preferably, the antibody for use as defined herein is glycosylated. In another preferred embodiment, the antibody for use as defined herein is deglycosylated. Preferably, by the deglycosylation the removal of *N*-glycans were removed from the antibody (schematically shown in Fig. 19). Deglycosylation can be obtained by different methods according to the invention. Fc-glycans can be deglycosylated, *e.g.,* by an enzymatic treatment with an endoglycosidase acting on complex type *N*-glycans or the antibody can be produced in a bacterial host such as *E. coli,* wherein no glycosylation has occurred.

As could be shown herein, the labeled-antibody-conjugates or labeled antibody fragments for use as defined herein allow detection of mSOD1 and mSOD1-aggregates by usual methods of immunoimaging *in vivo* (Examples 3-6 and, *e.g.,* Figs. 2, 3, 16). Therefore, preferably the labeled antibody or antibody fragment for use as specified herein is provided, wherein the imaging comprises PET imaging, SPECT imaging, CT imaging MRI, MPI, optical imaging, including fluorescence imaging, or PET/CT and SPECT/CT.

Early detection and quantification of mSOD1 and mSOD1-aggregates in a subject, due to the high sensitivity of immunoimaging methods such as PET, allows an early diagnosis of ALS, as well as tight monitoring of the progress of the disease and/or of the efficacy of a potential treatment. In the experiments described in the Examples below, preparation of a labeled antibody conjugate has been described and said conjugate validated as a sensitive radiotracer for immunoimaging of misfolded SOD1 (mSOD1). By the use of the conjugate in a method of diagnosis, ALS can be early diagnosed in a subject and a treatment started earlier than by a symptom-based diagnosis. In view of the data provided herein, the conjugate for use as defined herein allows a detailed monitoring of the disease state by visualizing mSOD1 and aggregates thereof even in the spinal motor neurons *in vivo* (see, *e.g.,* Figs. 3, 13 and 16), allowing thereby monitoring drug therapy and/or progression of ALS in the subject.

Therefore, the present invention also provides a drug for use in the treatment of ALS in patient who has been diagnosed for suffering or developing ALS and/or is monitored for efficacy of drug therapy and/or disease progression of ALS by a method as defined herein. Preferably the drug is selected hereby from the group consisting of Riluzole (Rilutek^{®}), Edaravone (Radicava^{®}), Tofersen (BIIB067), dextromethorphan HBr and quinidine sulfate (Nuedexta^{®}), AP-101, Reldesemtiv (CY5031), Arimoclomol, Levosimendan, Fasudil, pyrimethamine, (Daraprim^{™}), rapamycin, baclofen (Gablofen^{®}, Kemstro^{®}, Lioresal^{®}), diazepam (Diastat^{®}, Valium^{®}), amitriptyline (Elavil^{®}), trihexyphenidyl, Scopoderm^{®} (scopolamine patch), glycopyrrolate (Robinul^{®}), Ravulizumab (also known as BNJ441, ALXN1210, or Ultomiris^{®}) and Eculizumab (also known as Soliris^{®}).

As described in the examples (see Example 1), due to the experiments performed in connection with the present invention, a labeled antibody conjugate was produced and provided as defined herein when describing the conjugate envisaged for its intended use in a method of diagnosing amyotrophic lateral sclerosis (ALS) or monitoring drug therapy and/or progression of ALS in a subject, wherein the method comprises *in vivo* immunoimaging in a subject and the labeled-antibody conjugate comprises an antibody that specifically binds to misfolded SOD1 and a detection label conjugated to the antibody.

Therefore, in a further aspect, the present invention also provides a labeled antibody conjugate in all its preferred forms as defined hereinbefore.

Preferably, this labeled antibody conjugate or labeled antibody-fragment conjugate comprises:
(i) a variable heavy (VH) chain comprising VH complementary determining regions (CDRs) 1, 2, and 3, and/or a variable light (VL) chain comprising VL CDRs 1, 2, and 3, wherein
(a) VH-CDR1 comprises the amino acid sequence of SEQ ID NO: 3 or a variant thereof, wherein the variant comprises one or two amino acid substitutions,
(b) VH-CDR2 comprises the amino acid sequence of SEQ ID NO: 4 or a variant thereof, wherein the variant comprises one or two amino acid substitutions,
(c) VH-CDR3 comprises the amino acid sequence of SEQ ID NO: 5 or a variant thereof, wherein the variant comprises one or two amino acid substitutions,
(d) VL-CDR1 comprises the amino acid sequence of SEQ ID NO: 8 or a variant thereof, wherein the variant comprises one or two amino acid substitutions,
(e) VL-CDR2 comprises the amino acid sequence of SEQ ID NO: 9 or a variant thereof, wherein the variant comprises one or two amino acid substitutions, and
(f) VL-CDR3 comprises the amino acid sequence of SEQ ID NO: 10 or a variant thereof, wherein the variant comprises one or two amino acid substitutions; and/or
(ii) a VH chain and/or a VL chain, wherein
(a) the VH chain comprises the amino acid sequence depicted in SEQ ID NO: 1 or 2 or a variant thereof, wherein the variant comprises one or more amino acid substitutions; and
(b) the VL chain comprises the amino acid sequence depicted in SEQ ID NO: 6 or 7, or a variant thereof, wherein the variant comprises one or more amino acid substitutions; preferably wherein the VH and VL chain amino acid sequence is at least 90% identical to SEQ ID NO: 1 or 2 and 6 or 7, respectively.

More preferred, the labeled antibody conjugate or labeled antibody-fragment conjugate further comprises p-SCN-Bn desferoxamine (SCN-Bn-DFO) as the bifunctional chelator, and ⁸⁹Zr as the radionuclide tracer.

Particularly preferred, the labeled antibody conjugate is ⁸⁹Zr-DFO-AP-101 and comprises AP-101 as the antibody that specifically binds to misfolded SOD1, p-SCN-Bn desferoxamine (SCN-Bn-DFO) as the bifunctional chelator/linker and Zr-89 as the radionuclide.

As discussed above, antibodies usually occur in glycosylated forms. In a preferred embodiment, the antibody for uses as disclosed herein is glycosylated thus. However, as shown in the Examples and also discussed above, a deglycosylated tracer may be advantageous in immunoimaging due to its increased efficacy to detect mSOD1 aggregates also in tissues in which the glycosylated labeled antibody conjugate does not provide a signal, or only a weak signal. Therefore, in another preferred embodiment, the labeled antibody conjugate is deglycosylated. More preferred the labeled antibody conjugate in this embodiment is deglycosylated ⁸⁹Zr-DFO-AP-101 (Degly-⁸⁹Zr-DFO-AP-101). The exemplary preparation of such a conjugate is described in the Examples, Materials and Methods, subsection "Preparation of Degly-DFO-AP-101" below.

In a further aspect, the present invention also provides an antibody that specifically binds to misfolded SOD1, which antibody comprises a bifunctional chelator, which chelator preferably is p-SCN-Bn desferoxamine (SCN-Bn-DFO).

The antibody specifically binding to misfolded SOD1 preferably comprises:
(i) a variable heavy (VH) chain comprising VH complementary determining regions (CDRs) 1, 2, and 3, and/or a variable light (VL) chain comprising VL CDRs 1, 2, and 3, wherein
   (a) VH-CDR1 comprises the amino acid sequence of SEQ ID NO: 3 or a variant thereof, wherein the variant comprises one or two amino acid substitutions,
   (b) VH-CDR2 comprises the amino acid sequence of SEQ ID NO: 4 or a variant thereof, wherein the variant comprises one or two amino acid substitutions,
   (c) VH-CDR3 comprises the amino acid sequence of SEQ ID NO: 5 or a variant thereof, wherein the variant comprises one or two amino acid substitutions,
   (d) VL-CDR1 comprises the amino acid sequence of SEQ ID NO: 8 or a variant thereof, wherein the variant comprises one or two amino acid substitutions,
   (e) VL-CDR2 comprises the amino acid sequence of SEQ ID NO: 9 or a variant thereof, wherein the variant comprises one or two amino acid substitutions, and
   (f) VL-CDR3 comprises the amino acid sequence of SEQ ID NO: 10 or a variant thereof, wherein the variant comprises one or two amino acid substitutions; and/or
(ii) a VH chain and/or a VL chain, wherein
   (a) the VH chain comprises the amino acid sequence depicted in SEQ ID NO: 1 or 2 or a variant thereof, wherein the variant comprises one or more amino acid substitutions; and
   (b) the VL chain comprises the amino acid sequence depicted in SEQ ID NO: 6 or 7, or a variant thereof, wherein the variant comprises one or more amino acid substitutions;
preferably wherein the VH and VL chain amino acid sequence is at least 90% identical to SEQ ID NO: 1 or 2 and 6 or 7, respectively.

More preferred, the antibody specifically binding to misfolded and/or aggregated SOD1 is AP-101.

Preferably, the antibody (and consequently also the labeled antibody-conjugates as described herein) specifically binds misfolded and/or aggregated mammalian SOD1 such as mouse, rat or human misfolded SOD1, in particular preferred it binds human misfolded SOD1.

In view of the capability of the labeled antibody-conjugates as described herein to detect misfolded and/or aggregated SOD1 *in vivo* in a subject as described herein and shown in the Examples and in the Figures, the present invention in a further aspect relates to a diagnostic composition or kit comprising the labeled antibody conjugate as described herein, optionally further comprising a detection label. Preferably the antibody comprised in the diagnostic composition or kit is AP-101 or deglycosylated AP-101. Preferably the AP-101 antibody comprises a bifunctional chelator, which preferably is *p*-SCN-Bn desferoxamine (SCN-Bn-DFO).

The detection label comprised in said diagnostic composition or kit is selected as described above for the labeled antibody conjugate, labeled antibody-fragment conjugate. This means, depending on the intended use, preferably the label is selected from the group comprising or consisting of:
(a) for use in PET from: Fluorine-18 (F-18), Manganese-52 (Mn-52), Copper-64 (Cu-64), Gallium-67 (Ga-67), Gallium-68 (Ga-68), Antimony-117 (Sb-117), Scandium-43 (Sc-43), Scandium-44 (Sc-44), Titanium-45 (Ti-45), Bromium-76 (Br-76), Yttrium-86 (Y-86), Iodine-124 (I-124) and Zirconium-89 (Zr-89 or ⁸⁹Zr);
(b) for use in SPECT from: Indium-111 (In-111), Technetium-99m (Tc-99m), Iodine-123 (I-123), Iodine-131 (1-131), Lutetium-177 and Rhenium-186 (Re-186)
(c) for use in MRI and MPI: magnetic, paramagnetic or superparamagnetic ion complexes or particles, preferably containing Gaddolinum³⁺ (Gd³⁺), Manganese²⁺ (Mn²⁺); Copper ²⁺ (Cu²⁺); iron oxide (Fe₃O₄) contrast agents; and
(d) for use in optical imaging including fluorescent imaging from: fluorescein, Cy2, Cy3, Cy3B, Cy3.5, Cy5, Cy5.5, Cy7, Texas Red^{®} (Molecular Probes, Inc., Eugene, OR), AlexaFluor^{®} (Molecular Probes, Inc., Eugene), AlexaFluor 350, AlexaFluor 405, AlexaFluor 430, AlexaFluor 488, AlexaFluor 500, AlexaFluor 532, AlexaFluor 546, AlexaFluor 568, AlexaFluor 594, AlexaFluor 610, AlexaFluor 633, AlexaFluor 647, AlexaFluor 660, AlexaFluor 680, AlexaFluor 700, AlexaFluor 750, BODIPY FL, BODIPY R6G, BODIPY TMR, BOPDIPY 530/550, BODIPY 558/568, BODIPY 564/570, BODIPY 576/589, BODIPY 581/591, BODIPY 630/650, BODIPY 650/665, Rhodamine Green^{™}-X (Molecular Probes, Inc.), Rhodamine Red^{™}-X (Molecular Probes, Inc.), Rhodamine 6G, TMR, TAMRA^{™} (Applied Biosystems).

Preferably the detection label comprised in the diagnostic composition or kit is a radionuclide. More preferred it is a radionuclide selected from the group comprising or consisting of the radionuclides in items (a) to (c) above. Even more preferred, the diagnostic composition or kit is provided, wherein the detection label is a radionuclide selected from the group comprising or consisting of Fluorine-18 (F-18), Copper-64 (Cu-64), Gallium-67 (Ga-67), Gallium-68 (Ga-68), Antimony-117 (Sb-117), Scandium-43 (Sc-43), Scandium-44 (Sc-44), Titanium-45 (Ti-45), Indium-111 (In-111), Lutetium-177 (Lu-177) and Zirconium-89 (Zr-89 or ⁸⁹Zr). In particular preferred the detection label is a radionuclide, wherein the radionuclide is ⁸⁹Zr.

Preferably the labeled antibody conjugate or antibody comprised in the diagnostic composition or kit is ⁸⁹Zr-DFO-AP-101 or deglycosylated ⁸⁹Zr-DFO-AP-101 (Degly-⁸⁹Zr-DFO-AP-101).

The diagnostic composition or kit optionally further comprises instructions for use in a method of diagnosing amyotrophic lateral sclerosis (ALS) or monitoring drug therapy and/or progression of ALS in a subject wherein the method comprises *in vivo* immunoimaging of in subject and the labeled-antibody conjugate comprises an antibody that specifically binds to misfolded SOD1 and a detection label conjugated to the antibody as defined hereinabove.

In a further interrelated aspect, the present invention also refers to a method of *in vivo* immunoimaging of superoxide dismutase (SOD1) useful as a marker of amyotrophic lateral sclerosis (ALS) or as a marker for efficacy of drug therapy of ALS and/or progression of ALS in subject, comprising (a) administering a labeled-antibody conjugate as described herein to a subject; and (b) detecting the presence of the labeled-antibody conjugate in the subject *in vivo* by imaging.

Preferably the antibody which is used in this method of *in vivo* immunoimaging is an antibody which specifically binds to misfolded SOD1 as specified hereinbelow and further preferred, the subject is a human.

The antibody specifically binding to misfolded SOD1 which is administered in said method of *in vivo* immunoimaging preferably comprises:
(i) a variable heavy (VH) chain comprising VH complementary determining regions (CDRs) 1, 2, and 3, and/or a variable light (VL) chain comprising VL CDRs 1, 2, and 3, wherein
   (a) VH-CDR1 comprises the amino acid sequence of SEQ ID NO: 3 or a variant thereof, wherein the variant comprises one or two amino acid substitutions,
   (b) VH-CDR2 comprises the amino acid sequence of SEQ ID NO: 4 or a variant thereof, wherein the variant comprises one or two amino acid substitutions,
   (c) VH-CDR3 comprises the amino acid sequence of SEQ ID NO: 5 or a variant thereof, wherein the variant comprises one or two amino acid substitutions,
   (d) VL-CDR1 comprises the amino acid sequence of SEQ ID NO: 8 or a variant thereof, wherein the variant comprises one or two amino acid substitutions,
   (e) VL-CDR2 comprises the amino acid sequence of SEQ ID NO: 9 or a variant thereof, wherein the variant comprises one or two amino acid substitutions, and
   (f) VL-CDR3 comprises the amino acid sequence of SEQ ID NO: 10 or a variant thereof, wherein the variant comprises one or two amino acid substitutions; and/or
(ii) a VH chain and/or a VL chain, wherein
   (a) the VH chain comprises the amino acid sequence depicted in SEQ ID NO: 1 or 2 or a variant thereof, wherein the variant comprises one or more amino acid substitutions; and
   (b) the VL chain comprises the amino acid sequence depicted in SEQ ID NO: 6 or 7, or a variant thereof, wherein the variant comprises one or more amino acid substitutions;
preferably wherein the VH and VL chain amino acid sequence is at least 90% identical to SEQ ID NO: 1 or 2 and 6 or 7, respectively.

More preferred, the antibody specifically binding to misfolded SOD1 administered in the method of *in vivo* immunoimaging is AP-101.

Preferably the antibody which is administered to the subject in the immunoimaging method comprises a bifunctional chelator, which preferably is p-SCN-Bn desferoxamine (SCN-Bn-DFO).

Furthermore, the antibody is preferably labeled by a detection label, wherein depending on the intended use, the detection label is selected from the group comprising or consisting of:
(a) for use in PET from: Fluorine-18 (F-18), Manganese-52 (Mn-52), Copper-64 (Cu-64), Gallium-67 (Ga-67), Gallium-68 (Ga-68), Antimony-117 (Sb-117), Scandium-43 (Sc-43), Scandium-44 (Sc-44), Titanium-45 (Ti-45), Bromium-76 (Br-76), Yttrium-86 (Y-86), Iodine-124 (1-124) and Zirconium-89 (Zr-89 or ⁸⁹Zr);
(b) for use in SPECT from: Indium-111 (In-111), Technetium-99m (Tc-99m), Iodine-123 (I-123), Iodine-131 (1-131), Lutetium-177 and Rhenium-186 (Re-186)
(c) for use in MRI and MPI: magnetic, paramagnetic or superparamagnetic ion complexes or particles, preferably containing Gaddolinum³⁺ (Gd³⁺), Manganese²⁺ (Mn²⁺); Copper ²⁺ (Cu²⁺); iron oxide (Fe₃O₄) contrast agents; and
(d) for use in optical imaging including fluorescent imaging from: fluorescein, Cy2, Cy3, Cy3B, Cy3.5, Cy5, Cy5.5, Cy7, Texas Red^{®} (Molecular Probes, Inc., Eugene, OR), AlexaFluor^{®} (Molecular Probes, Inc., Eugene), AlexaFluor 350, AlexaFluor 405, AlexaFluor 430, AlexaFluor 488, AlexaFluor 500, AlexaFluor 532, AlexaFluor 546, AlexaFluor 568, AlexaFluor 594, AlexaFluor 610, AlexaFluor 633, AlexaFluor 647, AlexaFluor 660, AlexaFluor 680, AlexaFluor 700, AlexaFluor 750, BODIPY FL, BODIPY R6G, BODIPY TMR, BOPDIPY 530/550, BODIPY 558/568, BODIPY 564/570, BODIPY 576/589, BODIPY 581/591, BODIPY 630/650, BODIPY 650/665, Rhodamine Green^{™}-X (Molecular Probes, Inc.), Rhodamine Red^{™}-X (Molecular Probes, Inc.), Rhodamine 6G, TMR, TAMRA^{™} (Applied Biosystems).

The label is preferably covalently bound to the antibody via the bifunctional chelator or via a prosthetic group, wherein preferably the bifunctional chelator or prosthetic group is conjugated randomly on one or more lysine or cysteine residues of the antibody. More preferred, the bifunctional chelator or prosthetic group is conjugated randomly on oner or more lysine residues.

Preferably the detection label is a radionuclide. More preferred it is a radionuclide selected from the group comprising or consisting of the radionuclides in items (a) to (c) above. Even more preferred, the method of immunoimaging is provided, wherein the detection label is a radionuclide selected from the group comprising or consisting of Fluorine-18 (F-18), Copper-64 (Cu-64), Gallium-67 (Ga-67), Gallium-68 (Ga-68), Antimony-117 (Sb-117), Scandium-43 (Sc-43), Scandium-44 (Sc-44), Titanium-45 (Ti-45), Indium-111 (In-111), Lutetium-177 (Lu-177) and Zirconium-89 (Zr-89 or ⁸⁹Zr). Particularly preferred the detection label is a radionuclide, wherein the radionuclide is ⁸⁹Zr.

Preferably the labeled antibody conjugate or antibody used in the immunoimaging method is ⁸⁹Zr-DFO-AP-101 or deglycosylated ⁸⁹Zr-DFO-AP-101 (Degly-⁸⁹Zr-DFO-AP-101).

Preferably, the step of detecting the presence of the labeled-antibody conjugate in the subject *in vivo* by imaging comprises PET imaging, SPECT imaging, MRI, MPI, optical imaging including fluorescence imaging, or PET and SPECT, preferably in combination with CT (*i.e.,* PET/CT or SPECT/CT), mostly preferred the imaging comprises PET.

### Methods for Imaging

In one or more embodiments, methods for imaging are disclosed herein. In embodiments, methods for imaging include: (a) administering a labeled-antibody conjugate to a subject, wherein the labeled-antibody conjugate includes: an antibody that specifically recognizes and binds to mSOD1, and at least one detection label conjugated to the antibody, wherein the at least one detection label includes at least a label, preferably a radionuclide, a fluorophore, a magnetic/paramagnetic/supermagnetic particle, or a combination or any two or more thereof; and (b) detecting the presence of the labeled conjugate in the subject *in vivo* by imaging. In embodiments, the labeled-antibody conjugate is as described herein with regard to antibody conjugates. In further embodiments, the antibody and the at least one detection label are as described herein with regard to antibody conjugates.

Preferably, the methods for imaging include detecting the presence of the labeled conjugate in the subject *in vivo* by imaging. Preferably, the presence of the conjugate is detected in real time. Preferably, the presence of the labeled-antibody conjugate is detected non-invasively and/or minimally invasively.

According to aspects of the present disclosure, an antibody conjugate is an imaging agent which can be used to visualize mSOD1, such as in diagnostic procedures. Imaging can be performed by many procedures well-known to those having ordinary skill in the art, for example, by PET, SPECT, Cerenkov imaging, photoacoustic imaging, ultrasound imaging, optical coherence tomography, optical imaging, including fluorescence imaging, magnetic resonance imaging, magnetic particle imaging, bioluminescence imaging, or a combination of any two or more thereof.

In embodiments, the methods include administering a labeled-antibody conjugate to a subject.

In embodiments, the methods include administering an effective amount of a labeled-antibody conjugate to a subject. The labeled-antibody conjugate may be administered by any suitable route known to those of ordinary skill in the art. Preferably, administration of the labeled-antibody conjugate is by intravenous, intraarterial, intraperitoneal, intramuscular, subcutaneous, intrapleural, intrathecal, perfusion through a regional catheter, and/or direct intralesional injection. If the labeled-antibody conjugate is administered by injection, the administration may be by continuous infusion, by single bolus, and/or by multiple boluses. In some embodiments, administering the radiolabeled-antibody conjugate is nonimmunogenic to the subject. According to embodiments, administering a labeled conjugate to a subject includes administering a pharmaceutical composition including the labeled-antibody conjugate and a pharmaceutically acceptable carrier.

Preferably, the subject, as regards the diagnostic as the therapeutic aspects described therein, is a mammal. In some embodiments, the subject is a mammal selected from: humans, non-human primates, canines, felines, murines, bovines, equines, caprines, ovines, porcines, and lagomorphs. In a preferred embodiment, the subject is a rodent, including, but not limited to, a rat mouse, or guinea pig. In particular preferred, the subject is a mouse or a human.

In preferred embodiments, the labeled-antibody conjugate administered to a subject includes a radionuclide. Preferably, the labeled-antibody conjugate administered to a subject includes a labeled-antibody conjugate wherein the label is a radionuclide only, *i.e.* without a fluorophore or magnetic/paramagnetic/supermagnetic particle. In some embodiments, the labeled-antibody conjugate administered to a subject includes a labeled-antibody conjugate wherein the label is a fluorophore only, *i.e.,* without a radionuclide or para-/supermagnetic particle. In some embodiments, the labeled-antibody conjugate administered to a subject includes a labeled-antibody conjugate wherein the label is a para-/supermagnetic particle only, *i.e*., without a radionuclide or fluorophore.

In embodiments wherein the labeled antibody conjugate includes a fluorophore, the presence of the labeled-antibody conjugate may be detected *in vivo* by optical imaging. Various *in vivo* optical imaging techniques are known to those of ordinary skill in the art. (See *e.g.,* Ntziachristos, Annu. Rev. Biomed. Eng. 2006, 8:1-33; Troyan, S. L. et al., Ann. Surg. Oneal. 16, 2943-2952 (2009); Luker, G. D. & Luker, K. E., J. Nucl. Med. 49, 1-4 (2008); Tromberg, B. J. et al., Med. Phys. 35, 2443-2451 (2008), and their potential applicability to imaging-guided diagnostic and surgical methods has been proposed in several preclinical studies; Kirsch, D. G. et al., Nat. Med. 13, 992-997 (2007); von Burstin, J. et al., Int. J. Cancer 123, 2138-2147 (2008); and US Patent No. 9,409,923).

In embodiments, *in vivo* optical imaging is selected from confocal microscopy, planar imaging, fluorescence molecular tomography, complete projection tomography, fluorescence tomography direct imaging, two-photon *in vivo* imaging or a combination of any two or more thereof. In further embodiments, planar imaging is selected from epiillumination (*i.e.,* photographic) imaging, trans-illumination imaging, tomographic imaging, or a combination of any two or more thereof.

In embodiments wherein the presence of the labeled-antibody conjugate is detected by *in vivo* optical imaging, the fluorophore may be selected such that it emits fluorescence in the visible or near-infrared region. In illustrative, non-limiting embodiments, fluorophores emitting fluorescence in the visible or near-infrared region may be selected from fluorescein, Cy2, Cy3, Cy3B, Cy3.5, Cy5, Cy5.5, Cy7, Texas Red@ (Molecular Probes, Inc., Eugene, OR), AlexaFluor^{®} (Molecular Probes, Inc., Eugene), AlexaFluor 350, AlexaFluor 405, AlexaFluor 430, AlexaFluor 488, AlexaFluor 500, AlexaFluor 532, AlexaFluor 546, AlexaFluor 568, AlexaFluor 594, AlexaFluor 610, AlexaFluor 633, AlexaFluor 647, AlexaFluor 660, AlexaFluor 680, AlexaFluor 700, AlexaFluor 750, BODIPY FL, BODIPY R6G, BODIPY TMR, BOPDIPY 530/550, BODIPY 558/568, BODIPY 564/570, BODIPY 576/589, BODIPY 581/591, BODIPY 630/650, BODIPY 650/665, Rhodamine Green^{™}-X (Molecular Probes, Inc.), Rhodamine Red^{™}-X (Molecular Probes, Inc.), Rhodamine 6G, TMR, TAMRA^{™} (Applied Biosystems), or a combination of any two or more thereof.

In embodiments the presence of the labeled-antibody conjugate is detected by *in vivo* photoacoustic imaging. In illustrative, non-limiting embodiments, photoacoustic dyes included in the conjugate may be selected from Methylene blue, Evan's blue, Trypan blue, Patent blue, Indocyanine Green, IRDye800CW, DiR, Cy7, Cy7.5, and porphyrins or a combination of any two or more thereof.

In embodiments wherein the labeled antibody conjugate includes a combination of a radionuclide tracer and a fluorophore, the presence of the labeled-antibody conjugate may be detected *in vivo* by a combination of PET or SPECT and optical imaging.

In embodiments wherein the labeled antibody conjugate includes a combination of a radionuclide tracer and a fluorophore, the presence of the labeled antibody conjugate may be detected *in vivo* by a combination of PET or SPECT and photoacoustic imaging.

In embodiments the presence of the labeled-antibody conjugate may be detected *in vivo* by a combination of any two or more of: PET, SPECT, CT, Cerenkov imaging, photoacoustic imaging, ultrasound imaging, optical coherent tomography, magnetic resonance imaging, magnetic particle imaging, optical imaging, including fluorescence imaging, and/or bioluminescence imaging.

As described in Example 7 and shown in Fig. 22, the Experiments performed in the context of the present application for the first time shown the presence of misfolded and/or aggregated SOD1 in bones and joints of transgenic SOD1 G93A and also G37R mice, which are commonly used in the field as ALS models. Impairment of the bone function and structure, such as affected formation of osteoprogenitors, impaired osteoblast differentiation capacity, increased osteoclast formation and bone resorption leading to a decreased bone stability manifesting in osteopenia and fractures have been observed in ALS patients and ALS model organisms (Caplliure-Llopis at al. (2020), Zhu *et al.* (2015), Torres *et al.,* 2021 and Wang *et al.,* (2018)) confirming a relationship between muscles, which are affected in ALS by progressive atrophy and bones. The observations provided herewith, together with the findings described in WO 2012/080518 A1, that application of SOD1 targeting antibodies leads to an amelioration of ALS symptoms, such as slowing down the progressive motoric impairments, reduced loss of body weight in SOD1 transgenic animals and result in a delay of the disease onset, prolongation of survival and improvement of body weight and motor performance, strongly suggest that anti-SOD1 antibodies can be also of use in treatment and/or prevention of bone and/or joints related diseases, *e.g.,* by reducing and potentially preventing or delaying the onset of bone and/or joints deterioration in a disease associated with the presence or accumulation of misfolded and/or aggregated SOD1 in the bones and/or joints, *e*.*g*., in ALS.

Accordingly, in a further embodiment, the present invention provides an anti-SOD1 antibody or antigen binding fragment thereof for use in targeting misfolded and/or aggregated SOD1 in bone and/or joints of a subject, preferably wherein the antibody is the AP-101 antibody or an miSOD1 binding fragment thereof, or wherein the anti-SOD1 antibody is the labeled antibody conjugate as described herein.

Particularly preferred, the anti-SOD1 antibody or an antigen binding fragment thereof for use in targeting is provided for targeting of misfolded and/or aggregated SOD1 in subjects affected by or having ALS. Furthermore, preferably the subject is a mammal, mostly preferred a human.

### Terms

For the avoidance of any doubt, it is emphasized that the expressions "in some embodiments", "in a certain embodiments," "in certain instances," "in some instances," "in some aspects," "in a further embodiment," "in one embodiment," "in a further aspect," "in a first aspect," "in a second aspect," etc., and the like are used and meant such that any of the embodiments described therein are to be read with a mind to combine each of the features of those embodiments and that the disclosure has to be treated in the same way as if the combination of the features of those embodiments and aspects would be spelled out in one embodiment. The same is true for any combination of embodiments and features of the appended claims and illustrated in the Examples, which are also intended to be combined with features from corresponding embodiments disclosed in the description, wherein only for the sake of consistency and conciseness the embodiments are characterized by dependencies while in fact each embodiment and combination of features, which could be construed due to the (multiple) dependencies must be seen to be literally disclosed and not considered as a selection among different choices.

The terms "radionuclide", "radioactive nuclide", "radioisotope" or "radioactive isotope" are used interchangeably herein for a nuclide that has excess nuclear energy, making it unstable and wherein the excess energy is emitted, *e.g.,* as gamma rays and/or as particles. Preferably the emission takes place at least in part as particles such as beta particles (also called beta ray or beta radiation), mostly preferred as positrons.

The terms "fragment," "variant," "derivative" and "analog" when referring to antibodies or antibody polypeptides of the present invention include any polypeptides which retain at least some of the antigen-binding properties of the corresponding native binding molecule, antibody, or polypeptide. As indicated above, the variable region allows the antibody to selectively recognize and specifically bind epitopes on antigens. That is, the V_{L} domain and V_{H} domain, or subset of the complementarity determining regions (CDRs), of an antibody combine to form the variable region that defines a three-dimensional antigen-binding site. This quaternary antibody structure forms the antigen-binding site present at the end of each arm of the Y. More specifically, the antigen-binding site is defined by three CDRs on each of the V_{H} and V_{L} chains. Any antibody fragment which contains sufficient structure to specifically bind to misfolded SOD1 is denoted herein interchangeably as a "binding fragment" or an "immunospecific fragment." Antibodies or antigen-binding fragments, variants, or derivatives thereof and equivalent binding molecules of the invention include, but are not limited to, polyclonal, monoclonal, multispecific, human, humanized, primatized, murinized or chimeric antibodies, single chain antibodies, epitope-binding fragments, *e*.*g*., Fab, Fab' and F(ab')₂, Fd, Fvs, single-chain Fvs (scFv), single-chain antibodies, single domain antibodies, disulfide-linked Fvs (sdFv), fragments comprising either a V_{L} or V_{H} domain and fragments produced by a Fab expression library. ScFv molecules are known in the art and are described, *e.g.,* in US patent 5,892,019.

By "specifically binding", or "specifically recognizing", used interchangeably herein, it is generally meant that a binding molecule, *e.g.,* an antibody or fragment thereof binds to an epitope via its antigen-binding domain, and that the binding entails some complementarity between the antigen-binding domain and the epitope. According to this definition, an antibody is said to "specifically bind" to an epitope when it binds to that epitope, via its antigen-binding domain more readily than it would bind to a random, unrelated epitope. The term "specificity" is used herein to qualify the relative affinity by which a certain antibody binds to a certain epitope. For example, antibody "A" may be deemed to have a higher specificity for a given epitope than antibody "B," or antibody "A" may be said to bind to epitope "C" with a higher specificity than it has for related epitope "D". The reference that an antibody or fragment thereof binds to misfolded SOD1 as used in the present application is used in the meaning used for the term "specifically binding" or "specifically recognizing" as explained above. The term "binding" when referring to an antibody or fragment thereof as defined herein and its capability to bind misfolded SOD1 is used interchangeably and including the meaning of the terms "specifically binding" or "specifically recognizing" as defined above.

The term "detection label" refers to a chemical moiety that can be covalently attached to an antibody and that functions to provide a detectable signal. Examples of such labels include fluorescent moieties, chemiluminescent moieties, bioluminescent moieties, nanoparticles, magnetic particles, metal-containing particles, and radiolabels, such as radionuclides.

The term "bifunctional chelator" refers to a chemical moiety that attaches an antibody or antibody fragment to a label, *e.g.,* a radionuclide. Bifunctional chelators include 1) a chelator moiety functional to bind a (radionuclide) label and 2) a reactive functional group. Bifunctional chelators function by complexing a label *(e.g.,* radionuclide) with the chelator moiety and by covalently attaching the chelator moiety (and complexed radionuclide) to an antibody or antibody fragment via reaction of the reactive functional group with a corresponding reactive functional group of the antibody (or its fragment). Examples of suitable reactive functional groups capable of attaching a chelator moiety an antibody or fragment thereof, *e.g.,* to a primary amine group, a hydroxyl group, and/or a cysteine amino acid are known to those of skill in the art (see *e.g.,* Denardo et al., 1998, Clin Cancer Res. 4(10):2483-90; Peterson et al., 1999, Bioconjug. Chem. 10(4):553-7; and Zimmerman et al., 1999, Nucl. Med. Biol. 26(8):943-50).

The transgenic mice expressing mSOD1 are referred to interchangeably herein and in the Figures as Tg or ALS, wild type mice are referred to interchangeably herein as Wt or CTL.

By "subject", "individual" or "patient" is meant any subject, in particular a mammalian subject, *e.g.,* a human patient (*e.g.,* a patient having ALS) for whom diagnosis, prognosis, prevention or monitoring of the therapy is desired. The terms "subject", "individual" and "patient" are used interchangeable herein.

As used herein, the terms "treat" or "treatment" refer to both therapeutic treatment and prophylactic or preventative measures, in which the object is to prevent or slow down (lessen) an undesired physiological change or disorder, such as the development of cardiac deficiency. Beneficial or desired clinical results include, but are not limited to, alleviation of symptoms, diminishment of extent of disease, stabilized (*i.e.,* not worsening) state of disease, delay or slowing of disease progression, amelioration or palliation of the disease state, and remission (whether partial or total), whether detectable or undetectable. Those in need of treatment include those already with the condition or disorder as well as those prone to have the condition or disorder or those in which the manifestation of the condition or disorder is to be prevented.

Pharmaceutically acceptable carriers and administration routes can be taken from corresponding literature known to the person skilled in the art. The pharmaceutical compositions of the present invention can be formulated according to methods well known in the art; see for example Remington: The Science and Practice of Pharmacy (2000) by the University of Sciences in Philadelphia, ISBN 0-683-306472, Vaccine Protocols. 2nd Edition by Robinson et al., Humana Press, Totowa, New Jersey, USA, 2003; Banga, Therapeutic Peptides and Proteins: Formulation, Processing, and Delivery Systems. 2nd Edition by Taylor and Francis. (2006), ISBN: 0-8493-1630-8. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well-known conventional methods. These pharmaceutical compositions can be administered to the subject at a suitable dose. Administration of the suitable compositions may be affected in different ways. Examples include administering a composition containing a pharmaceutically acceptable carrier via oral, intranasal, rectal, topical, intraperitoneal, intravenous, intramuscular, subcutaneous, subdermal, transdermal, intrathecal, and intracranial methods. Aerosol formulations such as nasal spray formulations include purified aqueous or other solutions of the active agent with preservative agents and isotonic agents. Such formulations are preferably adjusted to a pH and isotonic state compatible with the nasal mucous membranes. Pharmaceutical compositions for oral administration, such as single domain antibody molecules (*e*.*g*., "nanobodies^{™}") etc. are also envisaged in the present invention. Such oral formulations may be in tablet, capsule, powder, liquid or semi-solid form. A tablet may comprise a solid carrier, such as gelatin or an adjuvant. Formulations for rectal or vaginal administration may be presented as a suppository with a suitable carrier; see also O'Hagan et al., Nature Reviews, Drug Discovery 2(9) (2003), 727-735. Further guidance regarding formulations that are suitable for various types of administration can be found in Remington's Pharmaceutical Sciences, Mace Publishing Company, Philadelphia, PA, 17th ed. (1985) and corresponding updates. For a brief review of methods for drug delivery see Langer, Science 249 (1990), 1527-1533.

Furthermore, unless stated otherwise, terms and expressions used herein in order to characterize the present invention are given in the definitions as provided in WO 2012/080518 A1, in particular in subsection "I. Definitions" at pages 10 to 30, the disclosure content of which is explicitly incorporated herein by reference. The same applies to the general embodiments disclosed in WO 2012/080518 A1 for antibodies, etc.

Several documents are cited throughout the text of this specification. The contents of all cited references (including literature references, issued patents, published patent applications as cited throughout this application including the background section and manufacturer's specifications, instructions, etc.) are hereby expressly incorporated by reference; however, there is no admission that any document cited is indeed prior art as to the present invention.

A more complete understanding can be obtained by reference to the following specific examples which are provided herein for purposes of illustration only and are not intended to limit the scope of the invention.

### EXAMPLES

The overall objective of the experiments described hereinbelow was to develop and validate a radiotracer derivatized from AP-101 and labeled with ⁸⁹Zr for detection of misfolded SOD-1 deposits via PET imaging. Such a radiotracer would find application in the early diagnosis of ALS, in the follow-up of the therapeutic response and/or in monitoring the disease, and as such be the first diagnostic tool available for ALS.

The specific aims of the experiments described herein below were:
**Aim-1** To prepare the ⁸⁹Zr-based PET tracers derivatized from AP-101 and to preferably to develop an automated process for the production of the selected ⁸⁹Zr-DFO-AP-101 PET tracer: Automation of ⁸⁹Zr-DFO-AP-101 using a cassette-based module will reduce dosimetry to the operator, allowing for reproducibility and enabling the GMP-compliant quality standards. Two ⁸⁹Zr-AP-101 derivatized PET tracers were developed; ⁸⁹Zr-DFO-AP-101 and the deglycosylated ⁸⁹Zr-DFO-AP-101 (Degly-⁸⁹Zr-DFO-AP-101) to reduce the uptake of the radioimmunoconjugate in nontarget tissues such as the liver and spleen by immune cells expressing Fc-γ-receptors (FcyR).

**Aim-2** To assess the efficacy of ⁸⁹Zr-DFO-AP-101 for early detection of aggregated SOD1 by PET imaging in an appropriate animal model. A longitudinal imaging study was performed to identify the optimal mice age and time post administration of ⁸⁹Zr-DFO-AP-101 for the detection misfolded SOD1 aggregation in symptomatic and pre-symptomatic transgenic (Tg, [B6.Cg - Tg(SOD1^{∗}G93A)1Gur/J)]) mice expressing the formation of m-SOD1 aggregate. Control imaging experiments were done with ⁸⁹Zr-DFO-AP-101 and Degly-⁸⁹Zr-DFO-AP-101 in wild type (Wt) mice.

**Aim-3** To determine if ⁸⁹Zr-DFO-AP-101 and Degly-⁸⁹Zr-DFO-AP-101 can be used for early diagnosis of ALS and in the follow-up of the therapeutic response; B) to estimate the anticipated human dose (OLINDA) of the selected ⁸⁹Zr-DFO-AP-101 PET tracer. A blocking study using unlabeled AP-101 (100 mg/Kg) was performed to evaluate the impact of unlabeled AP-101 on the bioavailability and the capacity of ⁸⁹Zr-DFO-AP-101 to bind to mSOD1 aggregates. A dosimetry studies in Wt mice was carried with Degly-⁸⁹Zr-DFO-AP-101 to estimate the expected injected dose in humans for diagnostic use with PET.

Hereinbelow, the preparation and the characterization (stability, shelf live) of ⁸⁹Zr-DFO-AP-101 and its analog Degly-⁸⁹Zr-DFO-AP-101 and the assessment of their potential for the detection of mSOD1 aggregates by PET imaging is described.

### Materials and Methods:

### Preparation of DFO-AP-101

Sample preparation. AP-101 (20 mg/mL, 1 mL) was centrifuged to remove all the additives using four 50 kDa molecular weight cut off centrifugal filter units (Amicon^{®} Ultra-05) and washed with PBS (trace metals basis, 300 µL each) pH=7.2 at 5000 rpm for 10 min four times. The final wash was done with NaHCO₃ 0.1 M, pH=8-9. The samples (-100-150 µL) were then combined in another 50 kDa molecular weight cut off centrifugal filter unit (Amicon^{®} Ultra-05) and centrifuged to reach a final volume of ~150-200 µL. The mAb AP-101 was diluted with a NaHCO₃ 0.1 M solution to reach a volume of -500-600 µL and transferred into a 5 mL screw thread vial (Fisherbrand) using a 200 µL micropipette. Concentrations lower than 2 mg/mL decreased the efficiency and the reproducibility of the conjugation reaction, accordingly, concentrations of AP-101 at 2 mg/mL and higher were used.

DFO Conjugation. The AP-101 solution (500-600 µL in NaHCO₃ 0.1 M) was incubated with three equivalents of SCN-Bz-DFO (15 µL of a solution of 1.1 mg of SCN-Bz-DFO dissolved in 100 µL of DMSO). The resulting mixture was kept for 1 hour at 37 °C. The DMSO concentration was kept below 2% to avoid change in the properties of AP-101. The conjugate was then purified ten times using the centrifugal filter technique described above using 2 centricons and PBS (trace metals basis, 200 µL each) pH=7.2. The samples (-100-150 µL) were then combined into a 5 mL screw thread vial (Fisherbrand) and stored at 4 °C. HPLC on Size Exclusion Chromatography (SEC) was performed on a BioSep-SEC S3000 column using standard mobile phase (0.1 M PBS+ 0.025% NaN₃) with a flow rate of 1 mL/minute at 280 nm. The determination of the average number of DFO per molecule of AP-101 was done using a modified isotopic dilution assay as described by Holland et al. J Nucl Med. (2010);51(8):1293-1300 in particular in Supplemental Material at pages 33/46, which document (including its Supplemental Material) is herewith incorporated herein in its entirety. Concentration of the conjugate was measured spectrophotometrically using the Bicinchoninic Acid (BCA) protein assay (He. F. (2011) Bio-101: e44. DOI: 10.21769/BioProtoc.44), which document is herewith incorporated herein in its entirety.

### Preparation of Degly-DFO-AP-101

To generate deglycosylated DFO-AP-101, the immobilized GlycINATOR spin columns containing the GlycINATOR enzyme covalently coupled to agarose beads GlycINATOR^{®} (EndoS2) was used for the removal of *N*-glycans. After equilibration of each column with 3 x 300 µL of 1M PBS (trace metal) and centrifugation at 200 g for 1 min, DFO-AP-101 (3 mg, 300 µL) was added to the column (0.5 mg per column). The volume of each column was adjusted to 300 µL of PBS and the solution is incubated at room temperature for 2 h by end-over-end mechanical mixing. After centrifugation at 1000 g for 1 min, Degly-DFO-AP-101 was collected in different reaction vials/tubes. For maximum yield, 300 µL of 1 M PBS was added to GlycINATOR spin columns followed by centrifugation for recovering residual Degly-DFO-AP-101, this step was repeated 3 times. After GlycINATOR digestion, HPLC on SEC was performed as described above to characterize the crude deglycosylated antibody. The conjugate was then purified three times using the centrifugal filter technique described above using 2 centricons and PBS (trace metals basis, 300 µL each) pH=7.2. The samples (-100-150 µL) were then combined in another 30 kDa molecular weight cut off centrifugal filter unit (Amicon^{®} Ultra-05) and centrifuged to reach a final volume of ~150-200 µL. The samples (~100-150 µL) were then combined into a 5 mL screw thread vial (Fisherbrand) and stored at 4 °C. Concentration of the conjugate was measured spectrophotometrically using the BCA protein assay (He. F. 2011). The determination of the average number of DFO per molecule of AP-101 was done using a modified isotopic dilution assay as described by Holland (Holland *et al.* 2010), which document is herewith incorporated herein in its entirety. The specific activity was determined following our previous reported procedure (Alnahwi *et al.* 2018), which document is herewith incorporated herein in its entirety.

### Radiolabeling and determination of the average number of chelators per antibody

Typically, 1-2 GBq of ⁸⁹Zr(oxalate)₂ were produced in a cyclotron facility following the procedure described at pages 2-9 of the article by Alnahwi et al., Appl. Sci. 8 (2018):1579 (Alnahwi *et al.,* 2018 - included herein in its entirety) and using ⁸⁹Y-pressed targets. ⁸⁹ZrCl₄ was loaded on the ion exchange cartridge and eluted as ⁸⁹ZrCl₄ in 0.5-1 mL of 1 M HCl with a 95% recovery yield. 400 µL of ⁸⁹ZrCl₄ (400-500 MBq) was neutralized with 110-120 µL of 2 M Na₂CO₃ to adjust the pH of ⁸⁹ZrCl₄ solution at 7-7.5. To the neutralized ⁸⁹ZrCl₄ solution, DFO-AP-101 (0.5-1 mg) in 0.5 M NaOAc pH=6.5 was added and the volume adjusted to obtain at least a concentration of 0.2 µg/µL of DFO AP-101 with a final pH of 7-7.2. The radiolabeling was allowed to progress for 45 min at 37 °C. The radiolabeling yield of the reaction was assessed by radio-TLC using 50 mM DTPA (pH 5-6) as mobile phase and counted on a TLC plate reader. Final radiochemical purity was determined using radio-TLC and SDS-PAGE.

### Stability studies

Shelf life of the PET tracers. Stability studies were performed on formulated ⁸⁹Zr-DFO-AP-101 and Degly-⁸⁹Zr-DFO-AP-101 (in 1M PBS, pH 7.2 alone or in the presence of gentisic acid (GA, 2,5 mg/mL) kept at 4 °C over 9-10 days. The presence of free ⁸⁹Zr was monitored by radio-TLC. The solution was analyzed by SDS-PAGE and exposed to autoradiography film to detect ⁸⁹Zr metabolites and/or aggregates.

*In vivo* stability studies. ⁸⁹Zr-DFO-AP-101 (175-200 MBq) in PBS were also incubated at 37 °C in 500 µL of fresh mouse plasma and PBS (1/1) for different periods of time. The stability was directly monitored by radio-ITLC using 50 mM DTPA as eluant to follow the presence of free ⁸⁹Zr.

### SDS-PAGE analysis

DFO-AP-101 and Degly-DFO-AP-101 were characterized via sodium dodecyl sulfate-polyacrylamide gel electrophoresis (SDS-PAGE). Briefly, 4-20 ng antibody (6-30 µL of a 0.6 mg/mL stock) were combined with 0-16 µL of Tris/glycine/SDS buffer and 4 µL 4X Laemmli protein sample buffer. For the denaturing conditions, 1µL of 1,4-dithiothreitol (DTT) was added and this mixture was then denatured by heating in boiling water for 5 min using a heat block. Subsequently, each sample was then loaded alongside an appropriate molecular weight marker (PageRuler^{™} Prestained Protein Ladder, ThermoFisher Scientific) onto a 4-15% precast polyacrylamide gel (Bio-Rad) and run for 1 h at 120 volts in Tris/Glycine/SDS buffer. The completed gel was stained using Coomassie Blue solution for 1 h, and destained overnight in destaining solution (10% acetic acid, 10% methanol). For ⁸⁹Zr-DFO-AP-101 and Degly-⁸⁹Zr-DFO-AP-101, the radioactive gel, was placed without coloration on a sheet of Saran wrap and exposed to a phosphor screen (Molecular Dynamics) for 30-60 min. The screen was then read by a Storm phosphor Imager (Amersham Biosciences). The image was analysed with Fiji software and the protein ladder picture from the gel was overlaid on the image for analysis.

### Animal care and experimental conditions

The animal study was approved by the University of Sherbrooke IACUC and was conform to the Canadian Council on Animal Care (CCAC) guidelines. Female Tg [B6.Cg - Tg (SOD1*G93A) 1Gur/J)] mice expressing the ALS genotype and their controls Wt [C56BL/6J] were purchased from Jackson (610 Main Street Bar Harbor, ME 04609). The mice were between 51 and 58 days old at arrival and were hosted at CIMS animal care facility until they were 126 days old. Upon arrival. The mice were examined to ensure that they were healthy and that each group was uniform in appearance and size. Throughout the study, the mice were housed by groups of maximum 4 mice per cage on a ventilated rack (Techniplast SealSafe Plus. Techniplast Ventilation Unit) in a controlled environment room. Cages were lined with a corn cob litter and included enrichment (Plastic dome. EnviroDri^{®} nesting material and wood block). Cages were changed once per week. Throughout the study the mice had free access to (standard tab) water and food (LabDiet 5008). Throughout the study the mice were observed daily to detect the expression of ALS symptoms (weight loss, suspension, and walking tests as described in Hatzipetros et al., J Vis Exp. 104 (2015):53257.

### Radiotracer administration

Under isoflurane anesthesia. (Induction 2-3% isoflurane, maintenance 1 - 2.5%, 1-1.5 L of oxygen/min) a catheter (30G needle/PE10 tube) was installed within the tail vein of the subject mouse for the administration of the radiotracer (20-30 MBq/0.2 mL saline 0.9%), either ⁸⁹Zr-DFO-AP-101, ⁸⁹Zr-DFO-AP-101 co-injected with 100 mg/kg of AP-101 or Degly-⁸⁹Zr-DFO-AP-101. Imaging days post administration of the radiotracer were optimized as the first results were collected the last groups being imaged only at days 7 and 10. Once the radiotracer administrated, the mouse was placed in its cage to recuperate from anesthesia and returned to the housing facility.

### PET imaging and image analysis

PET imaging was performed on days 7 and 10 following the administration of the radiotracer. Imaging was done on a LabPET-2 PET scanner (https://imagingrt.com/. Bore size: 72 mm. Axial FOV: 50 mm. Transaxial FOV: 60 mm. Spatial resolution: 0.74 mm (Isotropic at 5 mm from center). Detection efficiency (250-650 keV): 3.3% dedicated to small animal imaging. Preparation and PET imaging was done under isoflurane anesthesia delivered through a nose cone (Induction 2-3% isoflurane then 1 - 2.5%. 1-1.5 L of oxygen/min) the level of anesthesia being modulated if required during PET imaging. An ophthalmic gel was applied to the eyes to prevent eye dryness. The mouse was positioned within the field of view of the PET imager and a 30 min static acquisition was performed then the bed of the imager was moved within the CT module of the LabPET8 scanner, and a 2 min CT acquisition was performed. During PET imaging, the mouse was kept warm using pulsed hot air maintained at 32 °C, the environment temperature and respiration rate being monitored throughout the imaging procedure (PCSAM. Model 1025T Monitoring & Gating System, SA Instruments Inc.). Once the imaging procedure was completed, the mouse was placed in its cage to recuperate from anesthesia and returned to the housing facility.

### Aggregate identification and data processing

PET data were acquired in list mode and reconstructed on a 200 x 200 x 168 grid with 0.3 x 0.3 x 0.3 mm³ voxel size using 16 iterations and 8 subset of the 3D Ordered Subset Expectation Maximization (OSEM) algorithm. All PET images were corrected for the physical radionuclide decay of ⁸⁹Zr and random coincidences. Using PMOD software (version 3.8, PMOD Technologies Ltd., Zurich, Switzerland), the PET image of each animal was co-registered with its corresponding CT image by a rigid matching transformation.

The steps used to identify mSOD1 aggregates and quantify their radiotracer content are presented in Fig. 1. Once reconstructed the PET images were visually inspected to identify potential mSOD1 aggregates, which appear as discrete radiotracer spots within the spinal cord. If the spots were considered valid, a 3D region of interest (ROI) was drawn to include the whole mSOD1 spots. The criteria for validity were that the spot was well defined within the spinal cord and not contaminated by radioactivity spilled in form the presence of the radiotracer in the anterior portion of the vertebra. Once validated the radiotracer content of the mSOD1 spot was expressed in terms of %ID/g. The weight in gram of mSOD1 aggregates was estimated from the ROI volume assuming a density of 1g / ml. Next the spot radiotracer content was compared to that found in a similar ROI drawn within the same mouse (internal reference) and to an ROI traced in the same location in the control mice from the same group (external reference). From each experimental group (⁸⁹Zr-DFO-AP-101, ⁸⁹Zr-DFO-AP-101 blocked with 100m/kg AP-101 and Deglycosylated ⁸⁹Zr-DFO-AP-101), the following data were reported: Number of mice in the group, total number of spots identified, number of mice with spots, percent of mice with spots, number of spots per mouse, spots intensity (%ID/g), internal reference intensity (%ID/g), external reference intensity (%ID/g), spot to internal and external reference ratio. The mSOD1 spot to internal ratio is considered the most pertinent value as it represents the capacity to discriminate mSOD1 spots from their true background.

The average radiotracer content in the spinal cord and the anterior vertebra bone was measured by tracing thin ROI going through the length of these tissues (Fig. 2). From these profile ROI, the average %ID/g values measured within the spinal cord and the ventral vertebra bone were compared along with that of the "Spinal cord to Vertebra ratio". Other ROIs were drawn in the head of the femur and spleen to evaluate the average %ID/g values in these tissues following injection with ⁸⁹Zr-DFO-AP-101 and Degly-⁸⁹Zr-DFO-AP-101 (Fig. 3).

### Correction for spill in effect

Once located region of interest (ROI) was then traced around these spots and the measure radiotracer content corrected for the partial volume effect (PVE) using the recovery coefficient and for spill-in from the presence of free ⁸⁹Zr in the anterior part of the vertebra (Fig. 4). The recovery coefficient was obtained using the Ultra-Micro hot spot phantom (Fig. 5). The radioactivity content of m-SOD1 aggregated was expressed in terms of %ID/g.

### Biodistribution

After the last PET acquisition (Day 10), the mice were perfused to remove blood and the spinal cord and tissues of interest were removed to measure their radioactivity content. Under isoflurane anesthesia, the mouse was placed at a 45° angle. head up at the perfusion board set within blood collection dish. The thorax was then opened to get access to the heart, a catheter (25 G) was installed within the left ventricle to deliver the perfusion solution (PBS with heparin 10 U/mL) by means of a perfusion pump (20 mL @ 5 mL/min) and the right auricle was lacerated using a small scissor. The sampled tissues were rinsed with physiological saline, blotted dry and weighted. Their radioactivity content was then measured using a Hidex (HIDEX AMG, Gamble Technologies Limited, Mississauga, Canada) gamma counter. The measures were then corrected for radioactive decay. The results were expressed in units of percentage of injected dose per gram of tissue (%ID/g). The spinal cords and brains were preserved in (PFA 4% for 24 h and PBS for storage) and used for histopathological analysis.

### Example 1 - Synthesis and characterization of ⁸⁹Zr-DFO-AP-101 and Degly ⁸⁹Zr-DFO-AP-101

A large batch of DFO-AP-101 was prepared by optimizing the ⁸⁹Zr-chelator (DFO-SCN) conjugation on AP-101 lysine residues to obtain a DFO:AP-101 ratio of 1.1:1.0 (Table 1, n= 7). The ^{nat}Zr-DFO-AP-101 conjugate maintained a high selectivity and affinity (EC₅₀ = 0.5 nM) for misfolded SOD1 and similar to non-modified AP-101 (Fig. 6). The purity of DFO-AP-101 was confirmed by HPLC analysis where a narrow peak was observed for the conjugate (Fig. 7A). Absence of aggregates was confirmed by SDS-PAGE analysis (Fig. 7B). The radiolabeling yield was greater than 97% with an apparent specific activity of 0.66 GBq/mg.

**Table 1. Characterization of DFO-AP-101 and Degly-DFO-AP-101 (see also Fig. 6 in regard of EC₅₀ values)**

| **Compound** | **Ratio DFO:AP-101** | **Specific activity (GBq/mg)** | EC₅₀ (nM) (mSOD1) |
|---|---|---|---|
| DFO-AP-101 | 1.1 : 1.0 | 0.66 ± 0.11 (n=7) | 0.3 |
| Degly-DFO-AP-101 | 0.6:1.0 | 0.32±0.05 (n=2) | 2.4 |

To reduce the uptake of the radioimmunoconjugate in nontarget tissues such as the liver and spleen by immune cells expressing FcyR, the preparation of the deglycosylated analog of DFO-AP-101 was considered. The deglycosylation was performed on the DFO-AP-101 using EndoS2 kit (deGlycIT spin columns of the company Genovis AB, Sweden comprising a IgG-specific endoglycosidase acting on complex type N-glycans at the Fc-glycosylation site of IgG, wherein the endoglycosidase is covalently coupled to agarose beads). The loss of DFO chelator was observed during the deglycosylation: the resulting Degly-DFO-AP-101 has an average of 0.6 DFO per antibody as estimated by isotopic dilution techniques (Table 1). No trace of aggregate was observed on the purified Degly-DFO-AP-101 (Fig. 7B). The removal of glycans was monitored using gel electrophoresis under reducing conditions and confirmed by a downward shift in the mobility of the immunoglobulin heavy chains at ~55kD (Fig. 7B). High radiolabeling yield and apparent specific activity were achieved. Specific activity of ⁸⁹Zr Degly-DFO-AP-101 (0.32 ± 0.11 GBq/mg) was slightly lower than that of ⁸⁹Zr-DFO-AP-101. This can be explained by the lower DFO:AP-101 ratio. Degly-^{nat}Zr-DFO-AP-101 has a lower affinity (EC₅₀ = 2.4 nM) for misfolded SOD1 compared to non-modified AP-101, but its EC50 value is similar to that of ^{nat}Zr-DFO-AP-101

### Example 2 - Stability of Degly ⁸⁹Zr-DFO-AP-101

⁸⁹Zr-DFO-AP-101 is stable at 4 °C for over 10 days when formulated in PBS and for 7 days in mouse plasma. Only trace of free ⁸⁹Zr (< 5%) was detected after 10 days incubation in plasma. Degly-⁸⁹Zr-DFO-AP-101 was also stable at 4 °C in PBS with and without the presence of GA, which was used for its antioxidant and radioprotective properties (Joshi *et al.* 2012) (Fig. 9). One should note that the presence of ⁸⁹Zr-DFO chelator was mainly found on the immunoglobulin heavy chains at ~55kD of the Degly⁸⁹Zr-DFO-AP-101.

### Example 3 - Assessing the feasibility of using ⁸⁹Zr-DFO-AP-101 and Degly-⁸⁹Zr-DFO-AP-101 for PET imaging of spinal cord mSOD1 aggregates

A total of 47 mice (30 Tg and 17 Wt) were used for this study. The distribution of mice in the different experimental groups is presented in Fig. 10 along with the study timeline. The study was completed over several months and used different radiotracer productions. The longitudinal study was set to assess the optimal age and time post administration of ⁸⁹Zr-DFO-AP-101 to perform PET imaging. Tg and Wt mice aged 91, 110, 126 days old, were images at days 1, 4, 7 and 10 post administration of ⁸⁹Zr-DFO-AP-101 (Fig. 10).

From the results obtained, it was decided to set the age for PET imaging to 126 days old and limit PET imaging at days 7 and 10 post administration of ⁸⁹Zr-DFO-AP-101. It is also noteworthy that spots observed at day 7 were for most instance still present at day 10. The method used to analyze PET images was revised to consider the presence of radioactivity spill in onto the spinal cord from the presence of ⁸⁹Zr-DFO-AP-101 within the ventral portion of the vertebra seen in both the Tg and Wt mice.

At the time of PET imaging, the Tg mice were smaller as compared to the reference Wt population, which may be explained by their ALS phenotype. Indeed, the weight of the 126 days old Tg mice (25 ± 2 g) was significantly less than that of the Wt mice (30 ± 2 g), Fig. 11. The administrated doses of radiotracer normalized for mice weight (MBq/g) administrated to the Tg mice was significantly higher (0.69 ± 0.07 MBq/g) as compared to that received by the Wt mice (0.59 ± 0.06 MBq/g) Fig. 12. However, the specific activity of ⁸⁹Zr-DFO-AP-101 was high and highly reproducible and all administrated radiotracer doses were sufficient to obtain high quality PET images.

Although PET imaging was performed at day 7 and 10 post administration of the radiotracer the data analysis focused on the results from day 10 since this was the time where the highest uptake value of mSOD1 spots was observed (Fig. 12).

The number of detected spots including false positive spots was more important at day 10 as compared to day-7 for ⁸⁹Zr-DFO-AP-101 (6 spots at day-7 vs 18 spots at day-10,) and for Degly-⁸⁹Zr-DFO-AP-101 (6 spots at day-7 vs 12 spots at day-10). Among these, were some "false positive", these were either spots occurring in control mice or blocked mice. Except for one mouse, all the false (+) spots observed at day-7 were not seen again at day 10. The distribution of the m-SOD1 spots at day 10 within the mice spinal cord is presented in Fig. 13. All the spots found between the T10 and L2 vertebra, most of them being localized between the thoracic vertebra T11 - T13 (⁸⁹Zr-DFO-AP-101: 12/14, 85% vs Degly-⁸⁹Zr-DFO-AP-101: 9/11, 82%).

To assess the specificity of ⁸⁹Zr-DFO-AP-101, a group of mice (13 Tg, 4 Wt) were co-injected with 100 mg/kg of native AP-101 to block the binding of ⁸⁹Zr-DFO-AP-101 to mSOD1. The results are presented in Fig. 13 and 19. Co-injection with native AP-101 (blocking) considerably reduces the amount of mSOD1 spot detected, which decreased from 67% (6/9) with ⁸⁹Zr-DFO-AP-101 to 15% (5/13) under blocked conditions. The intensity of the spots was also decreased from 8.05 ± 1.30 %ID/g to 5.73 ± 0.28 with ⁸⁹Zr-DFO-AP-101 alone or in presence of AP-101 in excess. The ratio to internal reference remained similar for ⁸⁹Zr-DFO-AP-101 (4.62 ± 1.06) and blocked ⁸⁹Zr-DFO-AP-101 (4.70 ± 0.15).

⁸⁹Zr-DFO-AP-101 was found to accumulate in the vertebra ventral bone, which was problematic as the radiation from the vertebra at time would spill into the spinal cord masking potential mSOD1 spots. Degly-⁸⁹Zr-DFO-AP-101 was developed with the intent of reducing the non-specific uptake of the radiotracer in vertebra. The ability of ⁸⁹Zr-DFO-AP-101 and its deglycosylated analog for the detection of mSOD1 spots are compared in Fig. 13, 14 and 19.

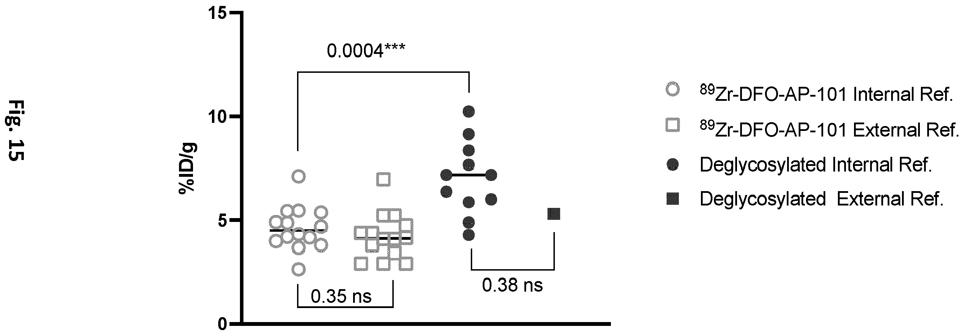

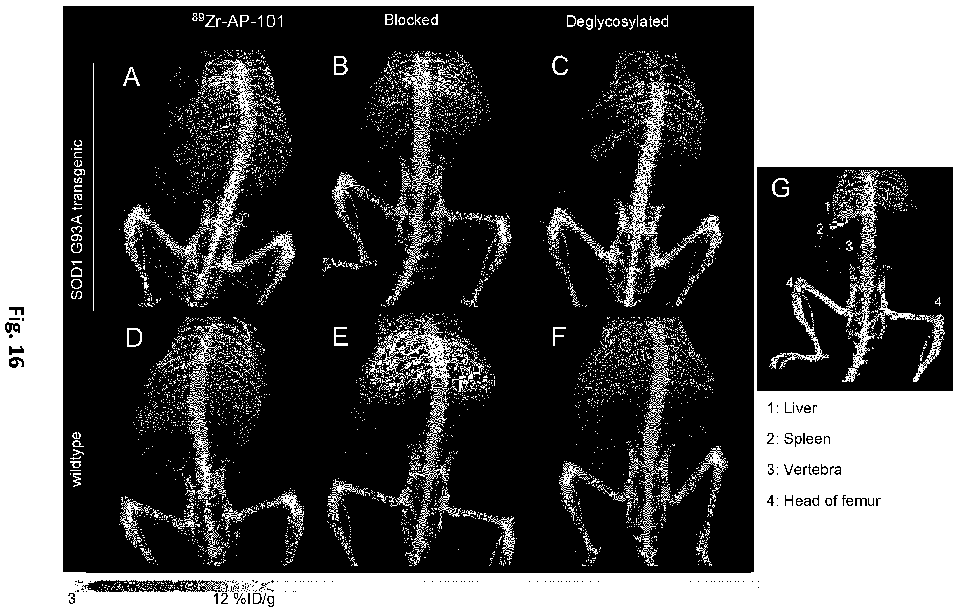

Assuming that all Tg mice had mSOD1 aggregates, deglycosylation increased the efficacy of the radiotracer at detecting mSOD1 aggregates from 67% (6 out of 9 mice) to 100% (7 out of 7 mice), (Fig. 14). Deglycosylation also significantly increased the average intensity of the detected spots from 8.05 ± 1.30 %ID/g for ⁸⁹Zr-DFO-AP-101 to 13.14 ± 2.80 %ID/g for Degly-⁸⁹Zr-DFO-AP-101 (Two-tails student-t test p = 0.01*). However, the spot to internal reference ratio was only slightly higher for Degly-⁸⁹Zr-DFO-AP-101 (1.92 ± 0.34) compared to ⁸⁹Zr-DFO-AP-101 (1.80 ± 0.40) and the difference was not statistically significant (Two-tail Student-t test p = 0.08 ns) (Fig. 15). The higher spinal cord background observed for Degly-⁸⁹Zr-DFO-AP-101 was likely due to its increased uptake into the vertebra (Fig. 16).

In summary, ⁸⁹Zr-DFO-AP-101 can specifically engage mSOD1 aggregate as demonstrated by co-injection of blocking quantities of native AP-101. The main limitation being that its accumulation in the ventral vertebra resulted in radioactivity spilling into the spinal cord potentially masking some mSOD1 spots. The presence of radioactivity in the vertebra was attributed to binding to FcyR, which prompted the development of deglycosylated ⁸⁹Zr-DFO-AP-101. Deglycosylation did not reduce the uptake of the radiotracer in the vertebra but resulted in a higher radiotracer uptake into mSOD1 aggregates.

### Example 4 - Spinal cord and vertebra uptake of ⁸⁹Zr-DFO-AP-101 and Degly-⁸⁹Zr-DFO-AP-101

The uptake of ⁸⁹Zr-DFO-AP-101 into the spinal cord and vertebra of the Wt an Tg mice was measured and compared for all experimental conditions. An overview of the uptake of ⁸⁹Zr-DFO-AP-101 in the vertebral column of the different groups of mice is given in Fig. 16.

It must be noted that the values measured in the spinal cord, even though a thin ROI was used, may for both the Tg and Wt mice be contaminated by radioactivity coming from the presence of radiotracer within the vertebra bone. Furthermore, for the Tg mice there is a possibility that the ROI might run through mSOD1 aggregates containing radiotracer thus increasing the average %ID/g value. To avoid these problems for some mice we also traced discrete ROI on area devoid of mSOD1 and spilled-in radioactivity.

The uptakes of ⁸⁹Zr-DFO-AP-101 and Degly-⁸⁹Zr-DFO-AP-101 in the Tg and Wt mice at day 10 post administration of the radiotracer are compared in Fig. 3 and 17. The concentration of ⁸⁹Zr-DFO-AP-101 in the spinal cord and vertebra of the Tg mice significantly exceeded that of the Wt mice (p = 0.01^{∗}), Fig. 17A-B and Fig. 3 E, F. However, both the Tg and Wt mice shared similar spinal cord to vertebra ventral bone ratio (p = 0,21) (Fig. 17C).

Blocking with 100 mg/kg of AP-101, significantly reduced the spinal cord (p = 0.0002^{∗∗∗}) and vertebra (p < 0.0001^{∗∗∗}) uptake of ⁸⁹Zr-DFO-AP-101 Fig. 17A-B. The spinal cord to ventral vertebra bone ratio was also significantly improved by blocking (p = 0.03^{∗}). In the Wt mice, blocking decreased the uptake of ⁸⁹Zr-DFO-AP-101 in the spinal cord, which was almost statistically significant (p = 0.053). However, blocking had no significant effect on either the vertebra uptake of ⁸⁹Zr-DFO-AP-101 (p = 0.16) or the spinal cord to vertebra ratio (p = 0.31) of Wt mice. Liver uptake was increased in both Tg and Wt mice, but the increase is more important in Wt mice under blocked conditions (Fig. 16).

In the experiments described herein, an accumulation of ⁸⁹Zr-DFO-AP-101 was observed in the anterior portion of vertebra. The accumulation of the radiotracer in the vertebra was at first attributed to the presence of free ⁸⁹Zr, this was later refuted and rather attributed to the binding of the heavy chain glycans of ⁸⁹Zr-DFO-AP-101 to immune cells FcyR (Vivier *et al.*,2019)*.* This prompted the development and *in vivo* validation of deglycosylated ⁸⁹Zr -DFO-AP-101 to decrease the *in vivo* off-target uptake (*i*.*e*., reduction of bone, liver and spleen uptakes), to improve target-to-healthy organ contrast and to get higher quality PET images.

In Tg mice, Degly-⁸⁹Zr-DFO-AP-101 showed a significant increase of the uptake of spinal cord (p < 0.0001^{∗∗∗}) and vertebra (p < 0.0001^{∗∗∗}) uptake but did not improve the spinal cord to vertebra ratio (p = 47) (Fig. 17 and Fig. 3 (continued 2)). Lower spinal cord and vertebra backgrounds were seen in Wt mice that received Degly-⁸⁹Zr-DFO-AP-101 resulting in better contrast between Wt and Tg mice. Our findings in Tg mice with Degly-⁸⁹Zr-DFO-AP-101 did little to support our initial hypothesis. Indeed, deglycosylation resulted in a sharp increase uptake of the radiotracer in the vertebra suggesting the presence of mSOD1 in bone or bone marrow and a specific uptake in these tissues.

### Example 5 - Head of the femur and spleen uptakes of ⁸⁹Zr-DFO-AP-101 and Degly-⁸⁹Zr-DFO-AP-101

The spleen and the head of the femur were readily visible on the PET images (Fig. 3) and their radiotracer content was found to vary with the experimental conditions. The average %ID/g values measured for ⁸⁹Zr-DFO-AP101 and Degly-⁸⁹Zr-DFO-AP101 within the spleen and the head of the femur are summarized in Fig. 3.

The head of the femur uptake mimicked what was observed for the vertebra bone uptake. Blocking in the Tg mice brought about a significant drop in the head of the femur uptake of ⁸⁹Zr-DFO-AP-101 (p< 0.0001^{∗∗∗}), while a significant increase of this uptake (p = 0.0004^{∗∗∗}) was observed when Degly-⁸⁹Zr-DFO-AP-101 was used for PET imaging (Fig. 3G).

Both ⁸⁹Zr-DFO-AP-101 in presence of AP-101 in excess and Degly-⁸⁹Zr-DFO-AP-101 resulted in a statistically significant decrease of the spleen uptake as compared to the uptake of ⁸⁹Zr-DFO-AP-101 (blocking p = 0.0003^{∗∗∗}; deglycosylation p < 0.0001^{∗∗∗}), Fig. 3H. One should note that the non-target tissue uptake of the tracers will contribute in increasing their radiation burden the impact of which will have to assessed by computing dosimetry.

### Example 6 - Biodistribution of ⁸⁹Zr-DFO-AP-101 and Degly-⁸⁹Zr-DFO-AP-101.

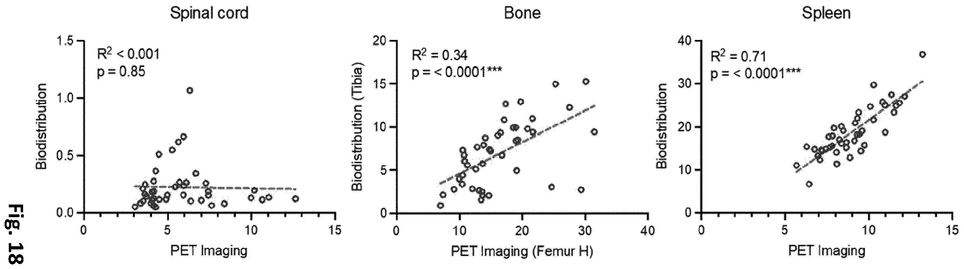

At day 10, after the last PET scan the mice were euthanized, and the tissues of interest removed after mouse perfusion, the residual activity (%ID/g) was measured using a gamma counter. The results for selected tissues are presented in Fig. 18, 19.

For the tissues visible on PET images, the %ID/g measured by PET was correlated with those measured by tissue sampling and counting. This was evaluated for the spinal cord, bone (head of the femur for PET vs tibia from biodistribution) and the spleen (Fig. 18). Except for the spinal cord where the tissue content in radiotracer was low, there was a significant correlation between the %ID/g values measured by PET imaging or biodistribution (bone: R²= 0.34, p < 0.0001^{∗∗∗∗} and spleen: R² = 0.71 p < 0.0001^{∗∗∗∗}).

At day-10, following iv administration of ⁸⁹Zr-DFO-AP101 in the Tg mice, the radiotracer was mostly concentrated in the spleen, blood, bone, and liver (Fig. 8). A similar biodistribution pattern was observed for the Wt mice but with a lower radiotracer tissue concentration level. This difference between the Tg and Wt mice is statistically significant for the blood (p = 0.05^{∗}), lungs (p = 0.03^{∗}) and muscle (p = 0.0007^{∗∗∗}).

Blocking with AP-101 significantly reduced the uptake of ⁸⁹Zr-DFO-AP-101 in most tissues, blood (p < 0.0001^{∗∗∗}), bone (p = 0.002^{∗∗}), spleen (p = 0.002^{∗∗}), lungs (p = 0.01^{∗}) and muscle (p = 0.05^{∗}), Fig. 8. However, blocking increased the uptake of ⁸⁹Zr-DFO-AP-101 in the liver (p< 0.0001^{∗∗∗}), this was more pronounced in the Wt mice (p = 0.0008^{∗∗∗}). These results are in accordance with PET image analysis (Fig. 16).

Degly-⁸⁹Zr-DFO-AP-101showed an increase of bone uptake (p = 0.09 ns) and significantly decreases in most other tissues such as blood (p = 0.0003^{∗∗∗}), spleen (p = 0.0002^{∗∗∗}), heart (p = 0.0008^{∗∗∗}), lungs (p = 0.0001^{∗∗∗}), kidneys (p = 0.01^{∗}) and muscle (p = 0.002^{∗∗}) when compared to those of ⁸⁹Zr-DFO-AP-101. Deglycosylation of ⁸⁹Zr-DFO-AP-101 had no significant impact on the liver radiotracer concentration.

### Example 7 - misSOD1 detection in cartilage tissue of bone and joints of SOD1 G93A transgenic mice.

Sections of dorsal and ventral vertebrae and from the joint area of the head of femur from SOD1 G93A transgenic and wild type mice have been stained with hematoxylin and with AP-101 and with a commercially available misSOD1 antibody (B8H10 from MÉDIMAPS, Gros-Louis et al., J. Neurochem., 113 (2010), 1188-1199) (See Fig. 22). Furthermore, a control staining was performed with 1nM isotype control antibody. No immunoreactivity could be detected with the isotype control antibody or in wildtype mice. In stainings of the SOD1 G93 transgenic mice misfolded SOD1 was detected with the AP-101 and the B8H10 antibody in both, the bone and joint cross sections.

### Summary of the experimental results

One long-term objective of the experiments described in Examples 1-6 was to validate the feasibility of using an ⁸⁹Zr-based AP-101 radiotracer for PET imaging of mSOD1 aggregates in ALS patients as an example of a tracer for immunoimaging of mSOD1 *in vivo* in a subject. The preclinical evaluation in Tg and Wt mice showed that it was feasible to use ⁸⁹Zr-DFO-AP-101 to identify discrete mSOD1 aggregates present in Tg mice spinal cord. However, a fair amount of ⁸⁹Zr-DFO-AP-101 was found in vertebra anterior bone portion. Spleen and some vertebra bone uptake was attributed to the binding of the heavy chain glycans of AP-101 to bone marrow T and B lymphocytes FcyR, which prompted the development of a deglycosylated analog of ⁸⁹Zr-DFO-AP-101.

Deglycosylation increased the efficacy of the radiotracer to detect mSOD1 aggregates from 67% to 100%. Both radiotracers were found to be able to engage spinal cord mSOD1 aggregates, the highest uptake and number of detected mSOD1 aggregates being obtained with Degly-⁸⁹Zr-DFO-AP-101. However, deglycosylation also significantly increased the uptake of the radiotracer in vertebra bone and the head of the femur suggesting the presence of mSOD1 in bone and cartilage tissue and a specific uptake in these tissues. Moreover, lower spinal cord and vertebra background was seen in Wt mice that received Degly-⁸⁹Zr-DFO-AP-101 resulting in better contrast between Wt and Tg mice with this ⁸⁹Zr-based AP-101 radiotracer. For these reasons, both radiotracers are suitable while Degly-⁸⁹Zr-DFO-AP-101 seems to be the most suitable radiotracer for clinical application. The presence of radioactivity in the vertebra will have less impact in humans as compared to mice, because of the differences in spine size and because of higher resolution and better capacity to discriminate lesions by clinical PET/CT.

Furthermore, detection of misfolded SOD1 in cartilage tissue of bones and, joints in two ALS animals models SOD1 G93A and SOD1 G37R transgenic mice indicates its potential role in the decrease of bone health observed in ALS patients and ALS model mice (Caplliure-Llopis *et al.,* (2020) and Zhu *et al.* (2015) and, in combination with previous findings regarding the potential of antibodies targeting the mutant or misfolded SOD1 in treatment of ALS related symptoms, *e.g*., extension of the lifespan of treated SOD1 transgenic animals, significant delay in the loss of their body weight and an amelioration of the motor neuron loss (see WO 2012/080518 A1, in particular in Example 9), indicates the potential of treatment with antibodies against pathologically misfolded/aggregated SOD1 species also in the treatment of bone and/or joint related diseases, in particular of diseases showing impairment of bone and/or joint function in connection with presence of misfolded SOD1 in the affected structures.

### REFERENCES

Abou DS, Ku T, Smith-Jones PM. In vivo biodistribution and accumulation of 89Zr in mice. Nucl Med Biol. 2011;38(5):675-681. doi:10.1016/j.nucmedbio.2010.12.011
Alnahwi AH, Tremblay S, Guérin B. Comparative Study with 89Y-foil and 89Y-pressed Targets for the Production of 89Zr. Appl. Sci., 2018, 8, 1579.
Alnahwi AH, Ait-Mohand S, Dumulon-Perreault V, Dory YL, Guérin B. Promising Performance of 4HMS, a New Zirconium-89 Octadendate Chelator. ACS Omega. 2020;5(19):10731-10739. Published 2020 May 5. doi:10.1021/acsomega.0c00207
Alnahwi AH, Tremblay S, Ait-Mohand S, Beaudoin JF, Guérin B. Automated radiosynthesis of 68Ga for large-scale routine production using 68Zn pressed target. Appl Radiat Isot. 2020;156:109014. doi:10.1016/j.apradiso.2019.109014
[ALSA] ALS Association resource page. Available at: http://www.alsa.org/about-als/facts-you-should-know.html.
Ayers JI, Fromholt SE, O'Neal VM, Diamond JH, Borchelt DR. Prion-like propagation of mutant SOD1 misfolding and motor neuron disease spread along neuroanatomical pathways. Acta Neuropathol. 2016;131(1):103-114. doi:10.1007/s00401-015-1514-0
Bensch F, Lamberts LE, Smeenk MM, et al. 89Zr-Lumretuzumab PET Imaging before and during HER3 Antibody Lumretuzumab Treatment in Patients with Solid Tumors. Clin Cancer Res. 2017;23(20):6128-6137. doi:10.1158/1078-0432.CCR-17-0311
Bidhendi EE, Bergh J, Zetterström P, Andersen PM, Marklund SL, Brännström T. Two superoxide dismutase prion strains transmit amyotrophic lateral sclerosis-like disease. J Clin Invest. 2016;126(6):2249-2253. doi:10.1172/JCI84360
Bosco DA, Landers JE. Genetic determinants of amyotrophic lateral sclerosis as therapeutic targets. CNS Neurol Disord Drug Targets. 2010;9(6):779-790. doi: 10.2174/187152710793237494
Caplliure-Llopis J, Escrivá D, Benlloch M, de la Rubia Ortí JE, Estrela JM, Barrios C., Poor Bone Quality in Patients With Amyotrophic Lateral Sclerosis., Front. Neurol. 11:599216. doi: 10.3389/fneur.2020.599216Eckelman WC, Kilboum MR, Joyal JL, Labiris R, Valliant JF. Justifying the number of animals for each experiment. Nucl Med Biol. 2007;34(3):229-232. doi: 10.10 16/j .nucmedbio.2007.0 1.005
Eckelman WC. Further discussions on choosing the number of animals for an experiment. Nucl Med Biol. 2008;35(1):1-2. doi:10.1016/j.nucmedbio.2007.10.002
Forsberg K, Jonsson PA, Andersen PM, et al. Novel antibodies reveal inclusions containing non-native SOD1 in sporadic ALS patients. PLoS One. 2010;5(7):e11552. Published 2010 Jul 14. doi:10.1371/journal.pone.0011552
Forsberg K, Andersen PM, Marklund SL, Brännström T. Glial nuclear aggregates of superoxide dismutase-1 are regularly present in patients with amyotrophic lateral sclerosis. Acta Neuropathol. 2011;121(5):623-634. doi:10.1007/s00401-011-0805-3
Gaudin E, Thibaudeau C, Arpin L, Leroux JD, Toussaint M, Beaudoin JF, Cadorette J, Paillé M, Pepin CM, Koua K, Bouchard J, Viscogliosi N, Paulin C, Fontaine R, Lecomte R. Performance evaluation of the mouse version of the LabPET II PET scanner. Phys. Med. Biol. 2021;66:065019.
Grad LI, Cashman NR. Prion-like activity of Cu/Zn superoxide dismutase: implications for amyotrophic lateral sclerosis. Prion. 2014;8(1):33-41. doi:10.4161/pri.27602
Graffmo KS, Forsberg K, Bergh J, et al. Expression of wild-type human superoxide dismutase-1 in mice causes amyotrophic lateral sclerosis. Hum Mol Genet. 2013;22(1):51-60. doi:10.1093/hmg/dds399
He, F. (2011). BCA (Bicinchoninic Acid) Protein Assay. Bio-101: e44. DOI: 10.21769/BioProtoc.44.
Heskamp S, Raavé R, Boerman O, Rijpkema M, Goncalves V, Denat F. 89Zr-Immuno-Positron Emission Tomography in Oncology: State-of-the-Art 89Zr Radiochemistry. Bioconjug Chem. 2017;28(9):2211-2223. doi:10.1021/acs.bioconjchem.7b00325
Holland JP, Divilov V, Bander NH, Smith-Jones PM, Larson SM, Lewis JS. 89Zr-DFO-J591 for immunoPET of prostate-specific membrane antigen expression in vivo. J Nucl Med. 2010;51(8):1293-1300. doi:10.2967/jnumed.110.076174
Joshi R, Gangabhagirathi R, Venu S, Adhikari S, Mukherjee T. Antioxidant activity and free radical scavenging reactions of gentisic acid: in-vitro and pulse radiolysis studies. Free Radic Res. 2012;46(1):11-20. doi: 10.3109110715762.2011.633518.
Maier M, Welt T, Wirth F, et al. A human-derived antibody targets misfolded SOD1 and ameliorates motor symptoms in mouse models of amyotrophic lateral sclerosis. Sci Transl Med. 2018;10(470):eaah3924. doi:10.1126/scitranslmed.aah3924
Menke-van der Houven van Oordt CW, McGeoch A, Bergstrom M, et al. Immuno-PET Imaging to Assess Target Engagement: Experience from 89Zr-Anti-HER3 mAb (GSK2849330) in Patients with Solid Tumors. J Nucl Med. 2019;60(7):902-909. doi: 10.2967/jnumed. 1 18.214726
Pandya DN, Bhatt N, Yuan H, Day CS, Ehrmann BM, Wright M, Bierbach U, Wadas TJ. Zirconium tetraazamacrocycle complexes display extraordinary stability and provide a new strategy for zirconium-89-based radiopharmaceutical development. Chem. Sci. 2017; 8:2309-2314.
Paré B, Lehmann M, Beaudin M, et al. Misfolded SOD1 pathology in sporadic Amyotrophic Lateral Sclerosis. Sci Rep. 2018;8(1):14223. Published 2018 Sep 21. doi:10.1038/s41598-018-31773-z
Tateno F, Sakakibara R, Kawashima K, et al. Amyotrophic lateral sclerosis presenting respiratory failure as the sole initial manifestation. Case Rep Neurol. 2014;6(2):213-216. Published 2014 Aug 14. doi:10.1159/000366191
Tokuda E, Takei YI, Ohara S, Fujiwara N, Hozumi I, Furukawa Y. Wild-type Cu/Zn-superoxide dismutase is misfolded in cerebrospinal fluid of sporadic amyotrophic lateral sclerosis. Mol Neurodegener. 2019;14(1):42. doi:10.1186/s13024-019-0341-5
Torres ML, Wanionok NE, McCarthy AD, Morel GR, Fernandez JM, Systemic oxidative stress in old rats is associated with both osteoporosis and cognitive impairment, Experimental Gerontology, 2021; 156 https://doi.org/10.1016/j.exger.2021.111596
Vivier D, Sharma SK, Adumeau P, Rodriguez C, Fung K, Zeglis BM. The Impact of FcyRI Binding on Immuno-PET. J Nucl Med. 2019;60:1174-1182. doi: 10.2967/jnumed.118.223636 Vu LT, Bowser R. Fluid-Based Biomarkers for Amyotrophic Lateral Sclerosis. Neurotherapeutics. 2017;14(1):119-134. doi:10.1007/s13311-016-0503-x
Wang, Z., Zhu, J. & Lu, H. Antibody glycosylation: impact on antibody drug characteristics and quality control. Appl Microbiol Biotechnol 104, 1905-1914 (2020). https://doi.org/10.1007/s00253-020-10368-7
Wang H, Yi J, Li X, Xiao Y, Dhakal K, Zhou J. 2018. ALS-associated mutation SOD1(G93A) leads to abnormal mitochondrial dynamics in osteocytes. Bone (2017); 106:126-138. doi:10.1016/j.bone.2017.10.010.
Zhu K, Yi J, Xiao Y, Lai Y, Song P, Zheng W, Jiao H, Fan J, Wu C, Chen D, Zhou J, Xiao G., Impaired Bone Homeostasis in Amyotrophic Lateral Sclerosis Mice with Muscle Atrophy, J Biol Chem. 2015 Mar 27;290(13):8081-94. doi: 10.1074/jbc.M114.603985. Epub 2015 Feb 3.

## Claims

1. A labeled-antibody conjugate for use in a method of diagnosing amyotrophic lateral sclerosis (ALS) or monitoring drug therapy and/or progression of ALS in a subject, wherein the method comprises *in vivo* immunoimaging of in subject and the labeled-antibody conjugate comprises:
(a) an antibody that specifically binds to misfolded SOD1; and
(b) a detection label conjugated to the antibody.

2. The antibody for use according to claim 1, wherein the detection label is a radionuclide.

3. The antibody for use according to claim 2, wherein the radionuclide is ⁸⁹Zr.

4. The antibody for use according to any one of the preceding claims, wherein the detection label is conjugated to the antibody with a bifunctional chelator or prosthetic group, preferably wherein the bifunctional chelator or prosthetic group is conjugated randomly on lysine residues of the antibody.

5. The antibody for use according to claim 4, wherein the bifunctional chelator comprises p-SCN-Bn desferoxamine (SCN-Bn-DFO).

6. The antibody for use according to any one of the preceding claims, wherein the antibody is AP-101.

7. The antibody for use according to any one of the preceding claims, wherein the antibody is glycosylated or deglycosylated.

8. The antibody for use according to any one of the preceding claims, wherein the imaging comprises positron emission tomography (PET) imaging, single photon emission computed tomography (SPECT) imaging, or combinations thereof with computed tomography (CT).

9. A drug for use in the treatment of ALS in a subject which (i) has been diagnosed for suffering or developing ALS and/or (ii) is monitored for efficacy of drug therapy and/or disease progression of ALS by a method as defined in any one of the preceding claims, preferably wherein the drug is selected from Riluzole (Rilutek^{®}), Edaravone (Radicava^{®}), Tofersen (BIIB067), AP-101, dextromethorphan HBr and quinidine sulfate (Nuedexta^{®}), Reldesemtiv (CY5031), Arimoclomol, Levosimendan, Fasudil, pyrimethamine, (Daraprim^{™}), rapamycin, baclofen (Gablofen^{®}, Kemstro^{®}, Lioresal^{®}), diazepam (Diastat^{®}, Valium^{®}), amitriptyline (Elavil^{®}), trihexyphenidyl, Scopoderm^{®} (scopolamine patch), glycopyrrolate (Robinul^{®}), Ravulizumab (BNJ441, ALXN1210, or Ultomiris^{®}) and Eculizumab (Soliris^{®})..

10. A labeled antibody conjugate as defined in any one of the preceding claims.

11. The antibody conjugate of claim 10, wherein the antibody conjugate is ⁸⁹Zr-DFO-AP-101 and comprises
(a) AP-101 as the antibody that specifically binds to misfolded SOD1;
(b) p-SCN-Bn desferoxamine (SCN-Bn-DFO) as the bifunctional chelator, and
(c) ⁸⁹Zr as the radionuclide.

12. An antibody that specifically binds to misfolded SOD1, which comprises a bifunctional chelator, preferably wherein the bifunctional chelator is p-SCN-Bn desferoxamine (SCN-Bn-DFO).

13. A diagnostic composition or kit comprising
- the labeled antibody conjugate of claim 10 or 11; or
- the antibody of claim 12, optionally further comprising a detection label, preferably comprising a radionuclide, preferably wherein the radionuclide is ⁸⁹Zr;
and optionally instructions for use in a method as defined in any one of claims 1 to 8.

14. A method of *in vivo* immunoimaging of superoxide dismutase (SOD1) useful as a marker of amyotrophic lateral sclerosis (ALS) or as a marker for efficacy of drug therapy of ALS in subject, comprising
(a) administering a labeled-antibody conjugate of any one of claims 10 to 12 to a subject; and
(b) detecting the presence of the labeled-antibody conjugate in the subject *in vivo* by imaging.

15. An anti-SOD1 antibody or equivalent SOD1 binding molecule for use in targeting misfolded and/or aggregated SOD 1 in bone and/or joints of a subject, preferably wherein the antibody AP-101.

16. The anti-SOD1 antibody or equivalent SOD1 binding molecule for use according to claim 15, wherein the anti-SOD1 antibody or equivalent SOD1 binding molecule is a labeled antibody conjugate as defined in any one of the preceding claims.

17. ⁸⁹Zr or ⁸⁹Zr-DFO for use in immunoimaging of misfolded and/or aggregated SOD1 in a subject, preferably for use in the labeled antibody conjugate of any one of the preceding claims and/or for use in early diagnostic of ALS and/or in the monitoring of therapeutic interventions of ALS.
